(19) Europäisches Patentamt
European Patent Office
Office européen des brevets

(11) Publication number: 0 290 246 A2

(12) # EUROPEAN PATENT APPLICATION

(21) Application number: 88304045.3

(22) Date of filing: 05.05.88

(51) Int. Cl.[4]: **A 61 K 39/205**
C 07 K 7/00, A 61 K 37/02,
A 61 K 39/385

(30) Priority: 08.05.87 US 47443

(43) Date of publication of application: 09.11.88 Bulletin 88/45

(84) Designated Contracting States: AT BE CH DE ES FR GB GR IT LI LU NL SE

(71) Applicant: THE WISTAR INSTITUTE OF ANATOMY AND BIOLOGY
36th & Spruce Streets
Philadelpia, PA 19104 (US)

(72) Inventor: Heber-Katz, Ellen
2300 Walnut Street
Philadelphia Pennsylvania 19103 (US)

Dietzschold, Bernhard
3430 Goshen Road
Newton Square Pennsylvania 19073 (US)

(74) Representative: Hale, Stephen Geoffrey et al
J.Y. & G.W. Johnson Furnival House 14/18 High Holborn
London WC1V 6DE (GB)

(54) **Vaccine for generating an immunogenic T cell response protective against rabies virus.**

(57) Vaccines which generate an immunogenic T cell response protective against a rabies virus disease state, comprise an immunologically effective amount of (1) a peptide-fatty acid conjugate, (2) a liposome composition and (3) an adjuvant. The conjugate (1) has the formula

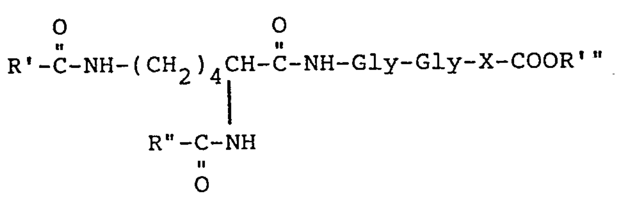

where R′ and R″ are alkyl groups containing 5 to 30 carbon atoms, and R‴ is selected from the group consisting of hydrogen and at least one amino acid residue, and X is an amino acid sequence corresponding to that of a peptide fragment derived from a protein of the virus which produces a T cell response, or a synthetic replica of said fragment.

EP 0 290 246 A2

**Description**

### Vaccine for Generating an Immunogenic T Cell Response Protective Against Rabies Virus

This invention relates to a vaccine for generating an immunogenic T cell response protective against rabies virus in the absence of antibody.

In our earlier application EP-A-203,676 we have disclosed and claimed a vaccine for generating an immunogenic T cell response protective against a virus, said vaccine comprising an immunologically effective amount of (1) a peptide-fatty acid conjugate, said peptide having an amino acid sequence corresponding to the sequence of a fragment of a protein or glycoprotein of said virus which produces a T cell response or a synthetic replica of said fragment, said conjugate having the formula

```
       O              O
       ‖              ‖
R'-C-NH-(CH2)4CH-C-NH-Gly-Gly-Peptide-Fragment-COOR'"
                 |
         R"-C-NH
            ‖
            O
```

where R′ and R″ are alkyl groups containing from 5 to 30 carbon atoms, and R‴ is selected from the group consisting of hydrogen and at least one amino acid residue; (2) a liposome composition comprising a mixture of phosphatidyl choline, cholesterol and lysophosphatidyl choline, and (3) an adjuvant.

That application is primarily concerned with vaccine against herpes virus, although several other viruses, including rabies are mentioned in passing.

Herpes viruses are widely spread in nature and natural hosts include fowl and animals, including man. Man is the natural host for herpes simplex viruses types 1 and 2 (HSV-1 and HSV-2, respectively), varicella/zoster, cytomegalovirus (CMV) and Epstein-Barr virus (EBV). Herpes simplex viruses are known to cause many diseases such as cold sores, encephalitis, eye infections, and genital infections, and HSV-2 has also been linked to cervical carcinoma. Clinical illness caused by herpes viruses presents a significant health problem and HSV-2 has produced major sociological consequences. At the present time no effective preventative measures are available.

There are experimental data, however, which indicate that immunization with a herpes virus or surface components of the virus can be protective. One such component, the virus envelope glycoprotein D molecule (gD), which is 59,000 MW, has been shown to be protective (Long, et al, Infect. and Immunity. 37: 761-764 (1984)). It has also been shown that antibody to this molecule can neutralize the virus and, when passively injected into animals, can be protective (Blalanchandran et al, Infect. and Immunity, 37: 1132-1137 (1982); Dix et al, Infect and Immun., 34: 192-199 (1981); Kapoor et al, J. Gen. Virol., 60: 225-233 (1982)). In all protocols for immunization against herpes virus thus published, high antibody titers have been reported. Therefore, it has been concluded generally that high antibody titers are extremely important in providing protection. Other studies indicate that such conclusion pertains to other virus infections as well.

It is known, however, that (1) recurrent herpes infections often occur in the presence of high antibody levels in the serum of patients and (2) individuals who have been infected previously with HSV-1 and are synthesizing antibody which crossreacts with both HSV-1 and HSV-2, nevertheless, contract HSV-2. More importantly, there have been studies showing not only the inability of antibody to protect, but that antibody can actually interfere with a protective host immune response (Wilson, et al, J. Immunology 132: 1552-1528 (1984); Babiuk et al, J. Microbiology, 25: 267 (1979)).

Recently, attention has been focused on internal antigens of viruses in the immune response to viral infection and immunization with particular reference to the priming of regulatory and cytotoxic T lymphocytes (CLTs) by delivering specific signals to the immune system (Townsend et al, Cell, 144: 959-968 (1986)); (Puddington, et al, J. Virol. 60: 708-717 (1986)); Celis, et al, J. Immunol. 136: 692-697 (1986)). One reason for focusing on the recognition by the immune system of internal rabies virus proteins, in contrast to the recognition of surface glycoprotein of rabies virus may not depend solely on the levels of virus-neutralizing antibodies induced by glycoprotein (Hattwick, et al, The Natural History of Rabies (Baer, G., ed.) Vol. 2, pp. 282-301, Academic Press, New York, London (1975)); Wiktor, et al, Proc. Natl. Acad. Sci. USA 75: 3938-3942 (1978)); Wiktor, et al, J. Exp. Med. 152: 99-112 (1980)); (Rupprecht, et al, Proc. Natl. Acad. Sci. USA 83: 7947-7950 (1986)). In a rabies virus infection or after immunization with live or inactivated rabies virus, evidence of the development of virus-induced CTL has suggested their possible role in cell-mediated immunity to rabies virus infection (Wiktor, et al, Proc. Natl. Acad. Sci., USA 74: 334-338 (1977b)); (Turner, Arch. Virol, 61: 321-325 (1979)); (Smith, Infect. Immun., 31: 297-308 (2981)) and that such CTL involvement was correlated with protection (Wiktor, et al, (1978) supra; Wiktor, et al, Rabies in the Tropics (Kuwert, et al, eds.), pp. 21-29, Springer-Verlag, Berlin (1985); Turner, Ann. Virol. (Inst. Pasteur) 136E: 453-460 (1985)). Recent demonstrations with vesicular stomatitis virus (VSV), another rhabdovirus, that the viral nucleocapsid protein

is the predominant cross-reactive antigen recognized by primary anti-VSV CTLs and that the nucleocapsid protein is a major target antigen for secondary stimulation of antiVSV CTLs (Puddington, et al, (1986), supra.; Yewdell, et al, J. Exp. Med. 163: 1529-1538 (1986)) have indicated that, as may be found in rabies virus, epitopes on the nucleocapsid protein are major determinants of the killing effect of anti-viral CTLs.

The nucleoprotein (N protein) and the nominal phosphoprotein (NS protein) [both are phosphorylated in rabies virus (Sokol et al, J. Virol., 7: 241-249 (1971); Dietzschold, et al, Virology, 98: 63-75 (1979))] represent a major proportion of antigen in the internal nucleocapsid (NC) complex of rabies virus (Schneider, et al, J. Virol., 11: 748-755 (1973); Sokol, et al, Proc. Natl. acad. Sci. USA 72: 933-936 (1975); Cox, et al, The Replication of Negative Strand Viruses (Bishop, D.H.L. and Compans, R.W., eds.), pp. 639-645, Elsevier/North-Holland, Inc., New York (1981); Wunner, et al, Immunochemistry of Viruses. The Basis for Seriodiagnosis and Vaccines (van Regenmortel, M.H.V. and Neurath, A.R., eds.), pp. 367-388, Elseiver Science Publishers B.V., Amsterdam (1985)). For some time, it has been recognized that the NC proteins of all rabies viruses share common antigenic determinants, as defined by cross-reactivity of polyclonal anti-NC antisera, but express qualitative antigenic differences as well (Sokol, et al, (1971), supra.: Tignor, et al, J. Gen. Virol., 37: 595-611 (1977)).

Recent analyses of numerous fixed (laboratory) and street (wildlife) strains of rabies virus using a panel of anti-NC monoclonal antibodies (MAbs) have further demonstrated that there are considerably greater degrees of antigenic variation than had been contemplated among NC (N and NS) proteins of rabies viruses and that the NC antigenic differences are often sufficient to distinguish viruses isolated from different animal species and from different geographical locations (Dietzschold, et al, Rev. Infect. Dis., to be published in Virus Research, (1987)).

Little is known about the nature, processing or function of specific epitopes of NC proteins of rabies viruses that are recognized by anti-NC antibodies, T helper cells, or CTL. To understand the immunological involvement of internal viral antigens requires a knowledge of the antigenic structure of the NC proteins. Using a panel of anti-NC MAbs, the distribution of antigenic sites on the rabies virus NC (N and NS) proteins was topographically described (Lafon, et al, J. Gen. Vir. 66: 2125-2133 (1985). Based on an antibody binding competition assay, five spatially distinct antigenic sites have been operationally defined; three on the N protein and two on the NS protein. Earlier studies had shown that the phosphorylation site(s) of N protein was confined to a trypsin-sensitive fragment, but the site(s) was never precisely located (Sokol, et al, Virology, 52: 246-263 (1973)). Recently, the amino acid sequences of the N and NS proteins of the Pasteur virus (PV) strain of rabies virus were deduced from the nucleotide sequence of genomic RNA (Tordo, et al, Nucleic Acids Res., 14: 2671-2683 (1986)). These amino acid sequences differ from the deduced sequences of the N and NS proteins of ERA strain rabies virus by 2 and 5 residues, respectively (Wunner, et al, Rev. Infect. Dis., in press (1987)).

In the report to be published in Virus Research, supra., are described the use of protein fragments and synthetic peptides to map antigenic domains of the ERA strain N and NS proteins within their deduced amino acid sequences and locate the phosphorylation site of the N protein within its primary sequence. Also described are the structure of two antigenic sites of the N protein and one antigenic site of the NS protein, and show that synthetic peptides resembling the structure of these antigenic sites are capable of inducing NC-specific antibodies in vivo and of stimulating proliferation of rabies antigen-primed T cells in vitro. These results suggest that certain continuous determinants for B cells are closely related to determinants recognized by immune T cells.

## Materials and Methods

### Preparation of Virus and Viral Nucleocapsid

The ERA strain of rabies virus was propagated on BHK 21 cell monolayers and purified as described (Wiktor, et al, J. Virol. 21: 626-635 (1977a)). Viral nucleocapsid was isolated and purified from rabies virus-infected BHK cells as described (Schneider et al, 1973, supra.).

For $^{32}P$-labeling of the viral proteins, cell culture medium was replaced 2 days after infection by phosphate-free minimum essential medium supplemented with $^{32}P$-orthophosphate (0.2 mCl/ml).

### Analysis and Preparation of N and NS Protein by SDS-Polyacrylamide Gel Electrophoresis (SDS-PAGE)

Electrophoresis of virus polypeptides was carried out in a discontinuous polyacrylamide gel system as described by Laemmli (Laemmli, Nature (London) 227: 680-685 (1970)). To visualize the protein in the gel, 10-20 μg of the virus dissolved in 0.05 M Tris-borate buffer, pH 7.8, containing 2% SDS was mixed with 20 μl 0.2 M borate buffer, pH 9.2, and 10 μl of a solution of 5 mg fluorescamine (Pierce Chemicals) in 1 ml DMSO. The mixture was incubated for 20 minutes at room temperature and the reaction quenched by adding 5 μl of 1 methanolamine. The derivatized protein samples were then combined with the untreated protein samples and subjected to SDS-PAGE. Protein bands were visualized by exposing the gel to UV light and excised with a razor blade. Excised protein bands were immersed for 5 minutes in $H_2O$ and then electroeluted overnight at a constant 80 V in 50 mM $NH_4HCO_3$ containing 0.1% SDS and 0.001 M dithiothreitol. The eluted protein was then precipitated with 5 volumes of ice-cold ethanol.

Trypsin Treatment of Rabies Virus Nucleocapsids

Purified nucleocapsids (500 µg) were dissolved in 1 ml of 0.1 M $NH_4HC)_3$. After addition of 5 µg of TPCK trypsin (Worthington) dissolved in 5 µl of 0.001 M HCl, the sample was incubated for 30 minutes at room temperature. Digestion was stopped by addition of 20 µg of soybean trypsin inhibitor (Sigma).

Cleavage of N and NS Protein with 3-bromo-3-methyl-2[(2-nitrophenyl)thiol]-3H-indole (BNPS-) skatole

Treatment of N or NS protein with BNPS-skatole was done essentially as described by Hunsiker et al (Hunsiker et al, Biochem. J., 187: 515-519 (1980)) (500 µg (≈ 10 nM) of N protein or 300 µg (≈ 10 nM) of NS protein was dissolved in 350 µl of 70% formic acid and, after addition of 3 µM of BNPS-skatole (Pierce), dissolved in 150 µl 88% formic acid. The mixture was incubated overnight with shaking in the dark at room temperature. After addition of 30 µM 2-mercapto-ethanol and further incubation for 5 hours at 37°C, the sample was dried with nitrogen and extracted three times with ethanol.

Cyanogen Bromide (CNBr) Cleavage of N and NS Protein

N or NS protein (10 nM) was dissolved in 500 µl 70% formic acid containing 100 mg CNBr. The solution was kept in the dark at room temperature for 18 hours. Reagents were then removed by repeated evaporation and resuspension in water.

Protease VA Digestion of N and NS Protein

Lyophilized N or NS protein (10 nM) was dissolved in 100 µl of 1% SDS. After addition of 900 µl 0.1 M $NH_4HCO_3$ and 10 µg protease VA, the sample was incubated for 16 hours at 37°C and then lyophilozed.

Purification of Protein Fragments by SDS-PAGE

Electrophoresis was performed in a 25% polyacrylamide separating gel with a 5% stacking gel. The gels were prepared as follows: (a) separating gel: 10 ml 1.5 M Tris sulfate, pH 8.4, 4.9 ml $H_2O$, 25 ml 40% acrylamide (acrylamide:bisacrylamide = 50:1), 60 µl 10% ammonium persulfate and 24 µl TEMED; (b) stacking gel: 3 ml 0.75 M Tris sulfate, pH 8.4, 7 ml $H_2O$, 2 ml 30% acrylamide (acrylamide:bisacrylamide = 20:1), 60 µl 10% ammonium persulfate and 5 µl TEMED. After polymerization, gels were left to stand for 48 hours.

Protein fragments were dissolved in a small volume of 6.5 mM Tris-borate buffer, pH 8.4, containing 1% SDS, 5% 2-mercaptoethanol and 10% glycerol. The gel was pre-electrophoresed at a constant 100 V for 2 hours. The peptide sample was then loaded on the gel and electrophoresis was continued for 16 hours at 5°C and a constant 100 V. Electrophoresis buffer was 6.5 mM Tris-borate, pH 8.6, containing 0.1% SDS, 1 mM dithiothreitol and 1 mM sodium thioglycolate.

Electrotransfer of Proteins and Peptides to Nitrocellulose Membranes

Electroblotting was carried out essentially as described by Towbin (Towbin et al, Proc. Natl. Acad. Sci. USA 76: 4350-4354 (1979)). Proteins or protein fragments were electrophoretically transferred to nitrocellulose paper (0.2 µm, Schleicher and Schuell) for 2 hours at 90 V and 5°C. The electrotransfer buffer was 25 mM Tris-HCl, pH 8.3, containing 20% methanol and 1 mM dithiothreitol.

After electrotransfer, the nitrocellulose membranes were rinsed with water and then immersed for 1 minute in a 0.1% solution of amido black in methanol-acetic acid-water (45:10:45) and briefly destrained with methanolacetic acid-water (45:10:45).

Elution of Peptides from Nitrocellulose Membranes

Preparative elution of peptides from nitrecellulose membranes was done as described by Parekh (Parekh et al, Analyt. Biochem., 148: 87-92 (1985)). The amido black-stained peptide bands were excised, cut into 1-mm squares and immersed for 15 minutes in a small volume of 50% pyridine in 0.1 M ammonium acetate, pH 8.9, containing 5 mM dithiothreitol. After centrifugation at 10,000 rpm for 10 minutes, the liquid phase was removed and lyophilized. The lyophilysate was then extracted three times with methanol. Between 15 and 20% of peptides applied to PAGE were recovered from nitrocellulose membranes.

Immunostaining of Electroblotted Peptides on Nitrocellulose Membranes

After electrotransfer, nitrocellulose membranes were washed three times with 50 mM Tris-HCl, pH 7.5, 0.15 M NaCl and then blocked with 2% normal horse serum in TPBS (0.1 M phosphate buffer, pH 7.5, containing 0.15 M NaCl and 0.05% Tween-20) for 30 minutes at room temperature. The blocked membranes were then washed three times with TPBS and incubated for 1 hour with MAb diluted in TPBS. Following incubation with MAb, the membranes were blocked again with 2% normal horse serum in TPBS for 30 minutes and then incubated for 1 hour with horseradish peroxidase-conjugated goat anti-mouse IgG complex (Cappel). Membranes were then washed three times with 50 mM Tris-HCl, pH 6.8, and incubated in freshly prepared substrate solution (10 ml 0.3% solution of 4-chloro-1-naphthol [Sigma] in methanol, 40 ml Tris-HCl, pH 6.8, and 50 µl 30% $H_2O_2$). Color development was allowed to proceed for up to 30 minutes until all bands were visible and the reaction was stopped by washing with water.

Detection of Proteins

One µl containing 250 ng protein or peptide was spotted on nitrocellulose membranes. Thereafter, the membranes were air-dried at room temperature and processed for immunostraining as described above for immunostaining of electroblots. Alternatively, the membranes were stained using the BIO RAD-Blot protein detection method. This method was also used when proteins or peptides were available only at sub-nanogram levels.

Protein Sequence Analysis

Dried peptides or proteins were dissolved in 88% formic acid and applied to a glass filter coated with polybrene. Automated Edman degradation of the peptides was performed on an Applied Biosystem 470 A gas-phase sequencer. PTH amino acids obtained from the sequencer were dried in vacuo, dissolved in 50 µl acetonitrile/water (1:1), and analyzed on a Zorbax PTH column as described (Glajch, et al, J. Chromatog., 318: 23-39 (1985)).

Analysis of Phosphorylated Amino Acids

To release phosphorylated amino acids, $^{32}P$-labeled N protein was dissolved in 2 N HCl and hydrolyzed for 8 hours at 110°C as described (Liu, et al, Biochemistry, 18: 3381-3386 (1979)). After lyophilization, the amino acids were derivatized with phenylisothiocyanate (PITC) and then analyzed using a PICO TAG column as described (Cohen, Biotechniques, 2: 273-276 (1984)). The separated $^{32}P$-labeled amino acids were identified based on the UV chromatogram of PITC phosphoserine and PITC phosphothreonine.

Peptide Synthesis

Peptide synthesis was done using the solid phase method described by Merrifield (Merrifield, Adv. Enzymol., 32: 221-296 (1969)). In a typical coupling reaction, the $^t$boc group of the amino terminus was removed with 50% trifluoracetic acid (TFA). After neutralization with N,N-diisopropylethylamine (DIEA), a 4- to 6- molar excess of preformed $^t$boc-amino acid-pentafluorophenylester (Kisfaludy, et al, Liebigs Ann. Chem., 1421-1429 (1973)) and a 1-molar equivalent of DIEA in dichloromethane (1:1) were added. After bubbling with $N_2$ gas for 1.5 to 2 hours, the resin was analyzed for the presence of free amino groups as described by Kaiser (Kaiser, et al, Anal. Biochem., 34: 595-598 (1970)). Couplings were repeated until less than 1% free $NH_2$ groups were found.

Coupling of Peptides to Protein Carriers

(a) Coupling via thiol groups. To couple peptides to protein carriers, the maleimide group was incorporated into the protein and the modified protein was allowed to react with a 20 molar excess of the peptide as described by Liu (Liu et al, 1979, supra.).

(b) Coupling via amino groups. One mg carrier protein and 1.5 mg peptide were dissolved in 2 ml 0.1 M phosphate buffer. The solution was stirred after dropwise addition of 1 ml of 0.25% glutardialdehyde, the mixture was stirred overnight and then dialyzed against 0.05 M $NH_4HCO$ 3 and lyophilized.

Preparation of Peptide Antisera

Female New Zealand rabbits were immunized subcutaneously with peptide coupled to keyhole limpet hemocyanin (KLH) in 50% complete Freund Adjuvant at three 2-week intervals. A total of 750 µg of peptide was used for immunization.

Indirect Immunofluorescence Assay

Antipeptide sera were heat inactivated at 56°C for 30 minutes and tested for specific activity by an indirect immunofluorescence technique. After fixatation with acetone, cells infected with various rabies virus strains were incubated with serial 3-fold dilutions of rabbit antisera and then with one conc. of fluoresceine-conjugated goat anti-rabbit IgG complex (Miles). Serum titers are given as the inverse of the last dilution showing a positive fluorescence.

Isolation of Rabies Virus-Reactive T Cell Lines and Clones

Peripheral mononuclear cells were obtained from rabies-vaccine recipients. Methods for T cell isolation and cell proliferation assays were recently described (Celis et al, 1986, supra.)

RESULTS

Electroblot Analysis of Rabies Virus Proteins with Nucleocapsid-Specific MAbs

Following SDS-page and electrotransfer to nitrocellulose membranes, N or NS protein showed reactivity with 6 of 35 anti-NC MAbs. Figure 1 shows the immunoreactivity pattern of rabies virus proteins, reduced with 2-mercapto-ethanol and electrotransferred with the six nucleocapsid-specific MAbs in Western blot analysis. Whereas MAbs 377-3, 802-2, 805-3 and 816-1 detected a prominent band corresponding to N protein, MABs 701-5 and 721-2 preferentially bound to NS protein. All six antibodies reacted with either N or NS protein, which was reduced and alkylated (data now shown), suggesting that the epitopes recognized by these antibodies are sequential in nature.

Reactivity of N Protein-Specific Antibodies with N Protein Fragments

As demonstrated in Fig. 2, MAbs 805-3 and 816-1 both recognized trypsin-treated nucleocapsid ribonucleoprotein (N-T), whereas MAbs 377-7 and 802-2 reacted only with N protein, suggesting that the epitopes recognized by the latter are located in the trypsin-sensitive terminal fragment. Automated Edman degradation of N and N-T proteins after purification by preparative SDS-PAGE and electroelution failed to reveal amino acid sequences, indicating that the $NH_2$-terminus of N as well as N-T protein was blocked. These experiments suggest that the trypsin-sensitive fragment of N protein which contains the epitopes for MAbs 377-7 and 802-2 is located at the COOH-terminus of the protein.

To further analyze the fine structure of epitopes, N protein was cleaved chemically at the tryptophan or methionine residues, or enzymatically at the glutamic acid residues. Following treatment of N protein with BNPS-skatole to obtain tryptophan terminal peptides, the fragments were separated on a 25% polyacrylamide gel and, after electrotransfer to nitro-cellulose paper, 12 peptide bands were visualized (Fig. 3). Lanes b-e of Fig 3 illustrate the reactivity pattern of fragments obtained after BNPS-skatole cleavage with N protein-specific MAbs. Whereas MAbs 805-3 and 816-1 reacted only with fragment N-W1, MAbs 337-7 and 802-2 both recognized fragments N-W1, N-W2, N-W3 and N-W4.

$NH_2$-terminal sequencing of the various fragments revealed that the $NH_2$-terminus of the N-W1 peptide was blocked; the first 10$NH_2$-terminal residues of peptide N-W3 matched residues 90-99 of N protein, and the first 13 $NH_2$-terminal amino acids of peptide N-W4 corresponded to residues 198-210 of the N protein (Table 1).

The reactivity pattern MAbs 377-7 and 802-2 with BNPS-skatole-cleaved fragments, together with the sequencing data obtained from these fragments, are consistent with the results obtained with trypsin-treated RNP (N-T), indicating that the epitopes for MAbs 377-7 and 802-2 are located in the COOH-terminal half of the N protein from residue 198.

After cleavage of N protein with CNBr, nine major bands and eight minor bands were resolved on a 25% polyacrylamide gel and could be visualized by employing the Biotin-Blot protein detection method after electrotransfer to nitrocellulose paper (Fig. 4, lane a). Only MAbs 377-7 and 802-2 reacted with the CNBr peptides N-CO and N-CI ((Fig. 4, lanes b and c). The $NH_2$-terminal sequence of peptides N-CO and N-CI was determined in order to map these peptides within the amino acid sequence of the N protein. The first 5 residues from the $NH_2$-terminus of peptide N-CO were found to match residues 339-343 (now shown) and the first 12 residues of peptide N-CI corresponded to residues 339 to 351 of N protein (Table 1). The sequence data indicate that peptide N-CI is nested within peptide N-CO and peptide N-CO is probably an incompletely cleaved fragment. However, if peptide N-CI represents a completely cleaved CNBr fragment, the sequencing data suggest that the epitopes for MAbs 377-7 and 802-2 are located in a region between methionine residues 338 and 411.

To identify the sites of N protein detected by MAbs 805-3 and 816-1, and also to further delineate the region recognized by MAbs 377-7 and 802-2, another set of fragments was prepared using protease VA. These fragments were separated on 25% polyacrylamide gel, and after electrotransfer to nitrocellulose paper, 13 bands were visualized by the Biotin-Blot protein detection method (Fig. 5, lane a). Fig. 5, lanes b and c show that both MAbs 377-7 and 802-2 recognized fragment N-V10 and to a lesser extent fragment N-V11. MAbs 816-1 (lane d) predominantly reacted with fragment N-V12 and to a lesser extent with fragments N-V8, N-V2 and N-V3. MAb 805-3 did not recognize any of the fragments obtained by protease V8 digestion of N protein (lane e).

Table 1 lists the $NH_2$-terminal sequence determined for peptide N-V10 and N-V12. The first 17 residues from the $NH_2$-terminus of peptide N-V10 correspond with residues 374 to 390 of the N protein. the $NH_2$-terminal sequence of peptide N-V10 enabled us to map the peptide within the N protein sequence and to delineate the region detected by MABS 377-7 and 802-2 as a sequence consisting of 19 amino acids. Fragment N-V12 produced two distinct residues at each degradation step, indicating that this band consists of two peptides. upon analysis and comparison with the deduced amino acid sequence of N protein, the presence of two different $NH_2$-terminal sequences was confirmed. The $NH_2$-terminal sequence of the peptide designated N-V12a corresponded with residues 184-195, whereas the second sequence of the peptide, designated N-V12b, matched residues 279 to 291 of N protein. The sequencing data obtained with peptide N-V12 suggest that the epitope for MAb 816-1 is located either in a region between residues 184 and 217 (V12a) or in a region between residues 279 and 337 (V12b).

Reactivity of NS Protein-Specific Antibodies with Fragments of the NS Protein

None of the cleavage fragments produced by treatment of NS protein with either BNPS-skatole or CNBr reacted with the NS-specific monoclonal antibodies 701-5 and 721-2. To obtain such fragments, the NS protein was digested with protease VA and analyzed for reactivity with these antibodies by SDS-PAGE and electrotransfer to nitrocellulose. Twelve bands were visualized with the Biotin-Blot protein detection method (Fig. 6, lane a). MABs 702-5 and 721-2 both reacted with fragment NS-V8 (lanes b and c). $NH_2$-terminal sequence of fragment V8 revealed a unique amino acid sequence which corresponded with residues 75-98 of the NS protein (Table 1). This finding suggests that the epitopes detected by MAbs 702-5 and 721-2 are located near the $NH_2$-terminus in a region between residues 75 and 98.

Mapping of the Phosphorylation Sites of the N Protein

The N protein was purified from rabies virus metabolically labeled with $^{32}P$-orthophosphate, and fragments of the $^{32}$-P-labeled N protein were generated by chemical or enzymatic cleavage methods. Autoradiography of $^{32}P$-labeled fragments produced by BNSP-skatole cleavage revealed that most of the radioactivity was associated with fragments N-W1, N-W3 and N-W4 (Fig. 3, lane f), whereas the only phosphorylated CNBr fragment of N protein was peptide N-C1 (Fig. 4, lane f). With $^{32}P$-labeled protease VA fragments of N protein, most of the radioactivity was associated with peptide N-V10. Finally, reverse-phase HPLC analysis of a trypsin digest of $^{32}$ P-labeled N protein revealed that $^{32}P$-activity eluted in a single peak. The $NH_2$-terminal amino sequence of the eluted radioactive material contained a unique amino acid sequence corresponding to residues 377-400 of N protein. The amino acid sequencing data of the phosphorylated peptides N-W3, N-W4, N-C1 and N-V10 (Table 1), as well as the amino acid sequence of the phosphorylated tryptic peptide, map the phosphorylation sites of the N protein to a region between residues 377 and 391. This region contains a threonine residue at positions 377 and 386 and a serine residue at position 389. After acid hydrolysis of $^{32}P$-labeled N protein, only phosphoserine was identified, indicating that serine in position 389 is the only phosphorylated amino acid of the N protein.

Antigenic Activity of Synthetic Peptides

To further analyze the structure of antigenic regions recognized by the MABs specific for N or NS proteins, a series of peptides were synthesized corresponding to the amino acid sequences of antigenically active cleavage fragments. These synthetic peptides were coupled to BSA and tested for binding activity with the MAbs. To determine the minimum number of amino acids required for binding of MAbs 377-3 and 802-2, overlapping peptides ranging in length from 10 to 19 amino acids and corresponding to amino acid residues 369 and 392 (Table 2) were synthesized. Results of immunoblot assay using MAbs 377-7, 802-2 805-3 and 816-1 and the different peptide antigens as shown in Fig. 7. MAbs 377-7 (lane b) and 802-2 (lane c), which reacted with native NC protein (Table 2), also recognized peptides N-V10c (369-383) and N-V10b (374-392). Peptide N-V10a (383-392) was not recognized by these antibodies, and no reactivity of peptides N-V10c, N-V10b and N-V10a was observed with MAbs 805-3 and 816-1. Together, these results indicate that the epitopes for MAbs 377-7 and 802-2 are located between residues 374 and 383. To determine which amino acids represent important recognition residues, peptides with substituted amino acids were synthesized. Whereas substitution of lysine 376 with leucine did not alter the binding of MAbs 377-7 and 802-2, substitution of threonine 377 with serine resulted in a significant decrease in the reactivity of MAb 377-7. Thus, theonine 377 is an important recognition residue.

Binding studies of MAb 816-1 with cleavage fragments of N protein mapped the epitope detected by the MAb between residues 184 and 217 or between 279 and 337. The reactivity of MAb 816-1 with synthetic peptide N-V12b, which corresponds to residues 184-203 but not with peptide N-V12a, which matches residues 313 to 337 (Table 2, Fig. 9, lane d), localized the epitope for this antibody to a region between residues 313 and 337.

The structure of the region of the NS protein recognized by MAbs 701-5 and 721-2 was confirmed by the reactivity of a synthetic peptide, corresponding to residues 75 to 90 of NS protein, with these MAbs (Fig. 10, lanes c and d, respectively).

T Cell Proliferation Induced by NC Protein-Derived Synthetic Peptides

We have investigated whether synthetic peptides that represent continuous determinant for B cells are also recognized by immune T cells. Human I1-2-dependent T cells which express the CD4 cell surface antigen and which were specific for rabies virus antigen were tested for their proliferative responses to purified NC protein and the NC protein-derived synthetic peptides N-V12b, N-V10c and NS-V8. As demonstrated in Table 3, not only N protein but also the synthetic peptides stimulated the response of rabies antigen-specific human T cells in the presence of irradiated autologous mononuclear cells. Human T cells specific for hepatitis B s-antigen did not proliferate in response to these peptides indicating that the response of rabies antigen-primed T cells to these synthetic peptides is antigen-specific.

Immunogenicity of Synthetic Peptides Corresponding to Antigenic Domains of the N an NS Proteins

To determine whether synthetic peptides corresponding to the antigenic region of N protein recognized by MAbs 377-7 and 802-2 mimic the immunogenic properties of the native nucleocapsid protein, peptides N-V10b and N-V10c were coupled to KLH by a C-terminal cysteine and used to raise antisera in rabbits. The reactivity of the antipeptide antisera with four MAbs was tested in an immunoblot assay (Fig. 7). Antisera raised to peptide N-V10b reacted with the corresponding peptide and also with peptides N-V10c and N-V10a (lane g), both of which have overlapping sequences with peptide N-V10b. Like MAbs 337-7 and 802-2, antisera raised to peptide N-V10b showed high level reactivity with native NC and denatured N protein (lane g), but did not recognize trypsin-treated NC (N-T) (row 3, lane g). Thus the antiN-V10b serum mimicked the reactivity of MAbs 377-7 and 802-2. The reactivity pattern of antiserum raised to peptide N-V10c (lane i) was similar to that obtained with anti-N-V10b serum, except that the reactivity with peptide N-V10a was low. The anti-N-V10c serum exhibits much higher binding titers with native NC ($>$1:1000) compared with the binding titers of anti-N-V10b serum ($\approx$ 1:500) or the titers obtained with MaBs 377-7 and 802-2 ($\approx$1:200).

Antisera produced against peptides N-V10b and N-V10c reacted with all rabies virus strains tested so far by

the indirect immunofluorescence staining technique. The reactivity of the anti-N-V10b serum (titer = 100) was much lower than that of the anti-N-V10c serum (titer > 2000). Whereas titers with the anti-N-V10b serum showed no variations among different rabies virus strains, the titer of the anti-N-V10c serum varied considerably (Mokola = 300, HEP = 2700).

As shown in Fig. 9, lane f, antisera raised against peptide N-V12b reacted with peptide N-V12b, but not with peptide N-V12a. In addition, the anti-N-V12b serum also reacted with native NC protein and trypsin-treated N protein (lane f). However, compared to the reactivity of the anti-N-V10c serum, the titer of the anti-N-V12b serum was low ($\approx 50$). Antiserum raised against peptide NS-V8 specifically reacted with peptide NS-V8 and NS protein. As tested in indirect immunofluoresence assay, the anti-NS-V8 serum recognized the rabies virus strain CVS, HEP, ERA and PM, but not the Mokola, Duvenhage 1 and Duvenhage 4 strains (Table 3). Thus, the reactivity pattern of the anti-NS-V8 serum parallels that obtained with MAbs 702-5 and 721-2.

Discussion

We have delineated epitopes on both the N and NS proteins of rabies virus nucleocapsid that are either highly conserved (MAbs 802-2, 816-1 and 805-3) or that vary (MAbs 377-7, 701-5 and 721-2) among rabies virus strains. Our immunological and chemical characterization of peptide fragments isolated following chemical or enzymatic cleavage of the N or NS proteins served to localize and to define the structure of these epitopes.

$NH_2$-termimal sequence analysis of the peptides recognized by MAbs 377-7 and 802-2 showed that the region involved in binding is located between amino acids 374 and 390 of the N protein. Analysis with synthetic overlapping peptides from 10 to 19 amino acids in length and covering amino acid resudes 369-392 further localized the region recognized by the two antibodies to residues 374-383. The observation that this small region is recognized by both MAbs 802-2 and 377-7 contrasts with the reported nonidentical reacivity pattern of these two MAbs with various rabies strains (Korowski, et al, 1985, supra.; Wiktor et al, 1985, supra.) This discrepancy may reflect the observed differences in the binding affinity of these two MAbs (M.Lafon, unpublished data); a single amino acid substitution in the N protein may then affect the weaker affinity binding of MAb 377-7 but not that of 802-2. Such is the case with the CVS-11 strain N protein, which contains a serine substitution at threonine 377 (Wunner et al, 1987, supra.) and which is recognized by MAb 802-2 but not by 377-7.

Analysis of N protein fragments revealed that only one cleavage fragment (N-V12) obtained by protase V8 digestion reacted with MAb 816-1. This fragment represents a mixture of two peptides; N-V12a, which could be mapped between residues 184 and 216, and N-V12b, which could be mapped between residues 279-337. Whereas a synthetic peptide corresponding to the first 20 N-terminal amino acids of peptide N-V12a showed no reactivity with MAb 805-3 or 816-1, the synthetic peptide corresponding to the last 24 C-terminal amino acids of N-V12b was highly reactive with MAb 816-1. Peptide N-V12b contains a cysteine residue at positions 317 and 333 and, in the native protein, these two cysteine residues may form a disulfide bridge resulting in a loop of 15 amino acids. The epitope corresponding to MAb 816-1 could be located in this loop. However, the reactivity of MAb 816-1 or 805-3 does not depend on an intact disulfide bridge, since both MAbs reacted with N protein that had been reduced and alkylated. That MAb 805-3 failed to react with N prrotein cleavage fragments or with any synthetic peptides corresponding to the N protein sequence was perhaps due to the relatively low binding affinity of this MAb. Competition binding experiments with N protein-specific MAbs 805-3 and 816-1 as well as the reactivity patterns of these two MAbs with the various rabies virus strains suggest that both antibodies recognize almost identical epitopes.

It was previously shown (Sokol et al, 1973, supra.) that the phosphorylation site of the rabies virus N protein was located at a terminal sequence. Our data, indicating phosphorylation of peptide fragments N-W1, N-W2, N-W3, N-C1, N-V10 and a tryptic fragment nested in the C-terminal region of the N protein, localized the phosphorylation site near the C-terminus between residues 388 and 390. Amino acid analysis revealed that only phosphoserine is present in the N protein, mapping the phosphate group to serine in position 389. Aspartic acid residues in positions 390, 391 and 393 and glutamic acid in position 392 indicate that the N protein phosphorylation site represents a casein-type phosphorylation site (Mamrack et al, 1979, supra.) The phosphorylated region of the N protein overlaps with the epitope recognized by MAb 802-1. This epitope, which is extremely acidic and hydrophilic, is conserved in all rabies virus strains analyzed so far, suggesting an association with some important biological functions of the virus. This region may be involved in the interaction of N protein with other rabies virus proteins such as NS or M proteins.

The epitopes corresponding to the NS-specific MAbs 701-5 and 721-2 were localized in the N-terminal half of the NS protein. Only the peptide obtained by protease V8 digestion and corresponding to amino acid residues 75 and 88 of the NS protein exhibited activity with MAbs 7012-5 and 721-2. CNBr fragments were not reactive with these two MAbs, suggesting that either the methionine residue at position 83 is an important recognition residue for these epitopes or that cleavage at this methionine residue results in reduced stability of both epitopes. Competition experiments with MAbs 701-5 and 721-2 indicated that the epitopes recognized by these antibodies are not identical. On the other hand, a relatively small peptide of 15 amino acids is recognized by both antibodies, suggesting the presence of two independent epitopes that we have been unable to separate. Because phosphorylation is associated with several peptides, and because only approximately 20% of the NS protein molecules appear to be phosphorylated (Dietzschold et al, 1979, supra.), a precise localization of phosphorylation sites on the rabies virus NS protein is difficult at present. These studies are now

in progress.

Antisera raised against synthetic peptides that resemble the binding sites of N- or NS-specific MAbs not only recognized specifically the corresponding peptides but also reacted with the native N or NS protein. Two of the peptide antisera, anti-N-V10b and anti-N-V10c, recognized all rabies virus strains tested so far. The reactivity pattern of these MAbs are identical to the pattern obtained with MAb 802-2. On the other hand, antiserum raised against peptide NS-V8 reacted only with certain rabies virus strains, and the reactivity pattern of this antiserum exactly paralleled th reactivity pattern of MAbs 702-5 and 721-2. We have previously reported that human T cells isolated from rabies vaccine recipients react with both the glycoprotein and NC proteins of the virus (Celis et al, 1986, supra.). In order to define regions of the NC proteins that are responsible for triggering T cell responses, we have tested synthetic peptides that represent continuous B cell determinants of NC proteins for their ability to stimulate the proliferation of rabies antigen-specific human T cells. Our data indicate that two continuous antigenic regions of the N protein and one continuous region at the NS protein which are recognized by mouse MAbs are also recognized by human T cells in an antigen-specific fashion. These results suggest that these three antigenic regions represent major immunogenic domains. We have recently demonstrated that a synthetic peptide which was derived from glycoprotein D of herpes simplex virus (HSV) 1 and 2 and which contained both B and T cell determinants can induce a protective immune response against a lethal HSV-2 infection (Watari et al, J. of Exper. Med., 165: 459 (1987)).

2. Objects of the Invention

It is an object of the invention to produce vaccines that offer significant protection for a long period of time against a large dose of virus, especially a herpes virus or rabies virus.

It is a further object to achieve such protection by a limited number of immunizations, in many instances a single immunization.

Another object of the invention is the development of an anti-HSV vaccine which utilizes a T cell response.

Still another object of the invention is the production of an anti-rabies vaccine which utilized a T cell response.

These and other objects of the invention will become further apparent from the detailed description of the invention, the appended claims, and the drawings in which

Figure 1 is a Western blot analysis of rabies virus polypeptides with MAbs to rabies virus nucleocapsid proteins. Rabies virus proteins were resolved by SDS-PAGE and electrotransferred to nitrocellulose membrane. Lanes a-f: enzyme immunostaining of rabies proteins using MAbs to rabies virus N or NS protein (lane a, MAb 377-7; lane b, MAb 802-2; lane c, MAb805-3; lane d, MAb 816-1; lane e, MAb 701-5; lane f, MAb 721-2).

Figure 2 is a western blot analysis of N protein (N) and trypsin-treated N protein (NT) with MAbs specific for N protein. N and NT were isolated as described in Materials and Methods. Equal amounts of N and NT were mixed and resolved on SDS-PAGE. After electrotransfer, the nitrocellulose membranes were immunostained using MAbs specific for N (lane b, MAb 377-7; lane c, MAb802-2; lane d, MAb 805-3, lane e, MAb 816-1). Lane a: N and NT were electrotransferred to nitrocellulose and visualized by the Biotin-Blot protein detection method.

Figure 3 is an analysis of fragments produced by chemical cleavage of N at the tryptophan residues. N protein labeled with $^{32}P$- was purified form SDS-PAGE and subjected t chemical cleavage with BNPS-skatole as described in the text. The resultant fragments were resolved on a 25% SDS-PAGE and then electrotransferred to nitrocellulose membrane. Lane a: electrotransferred fragments were visualized by the Biotin-Blot protein detection method. Lanes a-e: enzyme immunostaining of fragments with MAbs specific for N (b, MAb 377-7; c, MAb 802-2; d, MAb 805-3; e, MAb 816-1). Lane f: autoradiography of fragments produced from $^{32}P$-labeled N protein.

Figure 4 is an analysis of CNBr fragments of N protein. N protein labeled with $^{32}P$ was produced as described in the text and cleaved with CNBr. The resulting CNBr fragments were separated on 25% SDS-polyacrylamide gel and electrotransferred to nitrocellulose membranes. Lane a: CNBr fragments were visualized with the Biotin-Blot protein detection method. Lanes a-e: electrotransferred peptides were subjected to the enzyme immunostaining procedure employing MAbs specific for N (b, MAb 377-7; c, MAb 802-2; d, MAb 805-3; MAb 816-1). Lane f: autoradiography of CNBr fragments produced from $^{32}$-P-labeled N protein.

Figure 5 is an analysis of fragments produced by enzymatic digestion of N protein with protease V8. N protein labeled with $^{32}P$ was purified and digested with protease as described in the text. The resultant fragments were resolved on a 25% SDS-polyacrylamide gel and electrotransferred to nitrocellulose. Lane a: fragments were visualized with the Biotin-Blot protein detection method. Lanes b-d: enzyme immunostaining of electrotransferred fragments using MAbs specific for N (b, MAB 377-7; c, MAb 802-2; d, MAb 805-3; e, MAb 816-1). Lane f: autoradiography with fragments produced by protease V8 digestion of $^{32}P$-labeled N protein.

Figure 6 is an analysis of fragments produced by enzymatic digestion of the NA protein. NS protein from purified rabies virus was separated by SDS-PAGE and subjected to enzymatic cleavage with protease V8 as described in the text. The resultant fragments were resolved on a 25% polyacrylamide gel and electrotransferred to nitrocellulose membrane. Lane a: electroblotted fragments were visualized with the Biotin-Blot protein detection method. Lanes b-c: enzyme immunostaining of electrotransferred NS

peptide with MAbs (b, MAb 701-5; c, MAb 721-2; d, MAb 802-2).

Figure 7 is a dot blot analysis of synthetic peptides corresponding to the amino acid sequence of fragment N-V10. Antibodies: lane b, MAb 377-7; lane c, MAb 802-2; lane d, MAb 805-3; lane e, MAb 816-1; lane f, anti-N-V10b (prebleed); lane g, anti-N-V10b (immune serum); lane h, anti-N-V10c (prebleed); lane i, anti-N-V10c (immune serum). Lane a, antigens visualized by the Biotin-Blot protein detection method. Antigens: row 1, native RNP (50 ng); row 2, N protein eluted from SDS-PAGE (50 ng); row 3, trypsin-treated N protein eluted from SDS-PAGE (50 ng); row 4, synthetic peptide N-V10c coupled to BSA (1 μg); row 5, synthetic peptide N-V10b coupled to BSA (1 μg); row 6, synthetic peptide N-V10a coupled to BSA (1 μg).

Figure 8 is the effect of single amino acid substitutions on the antigenicity of peptide N-V10c. Antisera: lane b, MAb 377-7; lane c, MAb 802-2; lane d, MAb 805-3; lane e, MAb 816-1; lane a, antigens visualized by the Biotin-Blot protein detection method. Antigens: row 1, N protein eluted from SDS-PAGE (50 ng); row 2, fragment N-V10 eluted form SDS-PAGE (μ100 ng); row 3, synthetic N-V10b (1 μg); row 4, synthetic peptide N-V10c-L (1 μg); row 5, synthetic peptide N-V10c-T (1 μg); row 6, synthetic peptide N-V10c-S (1 μg).

Figure 9 is a dot blot analysis of synthetic peptides corresponding to amino acid sequences of fragments N-V12a and N-V12b. Antibodies: lane b, MAb 802-2; lane c, MAb 805-3; lane d, MAb 816-1; lane e, anti-N-V12b (prebleed); lane f, anti-N-V12b (immune serum); lane a, antigens visualized by the Biotin-Blot protein detection method. Antigens: row 1, RNP (50 ng); row 2, trypsin-treated N protein eluted from SDS-PAGE (50 ng); row 3, synthetic peptide N-V12b, row 4, synthetic peptide N-V12a.

Figure 10 is a dot blot analysis of synthetic peptides corresponding to amino acid sequences of fragment NS-V8. Antibodies: lane b, MAB 802-2; lane c, MAb 701-5; lane d, MAb 721-2; lane e, anti-NS-V8 (prebleed); lane f, anti-NS-V8 (immune serum); lane a, antigens visualized by the Biotin-Blot protein detection method. Antigens: row 1, N protein eluted from SDS-PAGE; row 2, NS protein eluted from SDS-PAGE; row 3, synthetic peptide NS-V8.

Figure 11 is a plot of the percentage of normal mice vs. time (days) comparing the protection against lethal infection by HSV-2 provided by the vaccine of the present invention compared to certain control inoculants;

Figures 12a and 12b show the antibody binding activity induced by the HSV vaccine of the invention in relation to certain control inoculants, and

Figure 13 is similar to Fig. 11, but using a reduced concentration of herpes virus ($4LD_{50}$ vs. $10LD_{50}$) in the challenge;

Figure 14 is a plot of the percentage of normal mice vs. time (days) comparing the protection against lethal infection by HSV-2 provided by a herpes vaccine of this invention compared to a control inoculant and another herpes vaccine of the invention, the difference being in the adjuvant used.

Summary of the Invention

The vaccines of the present invention have been found to generate a T cell response protective against a virus, such as a herpes or rabies virus, and comprise an immunologically effective amount of (1) a peptide-fatty acid conjugate, the peptide having an amino acid sequence corresponding to the sequence of a fragment of a glycoprotein or protein of the virus which produces a T cell response or a synthetic replica of the glycoprotein or protein fragment, the conjugate having the formula

$$R'-\overset{\overset{O}{\|}}{C}-NH-(CH_2)_4\underset{\underset{R''-\underset{\underset{O}{\|}}{C}-NH}{|}}{CH}-\overset{\overset{O}{\|}}{C}-NH-Gly-Gly-Peptide-Fragment-COOR'''$$

where R′ and R″ are alkyl groups containing from 5 to 30, preferably from 10 to 20 carbon atoms, and R‴ is selected from the group consisting of hydrogen and at least one amino acid residue; (2) a liposome with a composition comprising a mixture in the preferred vaccine of phosphatidyl choline, cholesterol and lysophosphatidyl choline, and (3) an adjuvant.

As used in the specification and appended claims, the expression "T cell response" refers to the ability of T cells to respond to antigen by production of lymphokines and/or molecules involved in effector functions, other than help for B cells in production of antibody, after antigen stimulation. Tests have shown that this vaccine does not induce antibody and directly relates to the phenomenon of "immune enhancement" in which antibodies enhance viral infectivty (Hawks, et al, J. Viro. 33: 250 (1967); Halstead et al, J. of Immun. 132: 1526 (1984)), this has been shown to be especially important in viruses that infect macrophages (i.e. HTLV-3).

A preferred vaccine is an anti-HSV-1 or HSV-2 vaccine in which the immunological active peptide-fatty acid conjugate has an amino acid sequence corresponding to a fragment of herpes simplex virus type 1 envelope glycoprotein gD-1 or herpes simplex virus type 2 envelope glycoprotein gD-2 which produces a T cell response or a synthetic replica of such glycoprotein fragment, the conjugate having the formula

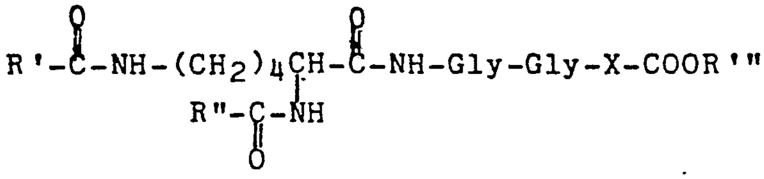

where R′, R″ and R‴ are stated above, and X is an amino acid sequence corresponding to that of a fragment of herpes virus envelope glycoprotein, components (2) and (3) of the vaccine being as previously indicated. In a particularly preferred vaccine of the invention X has the amino acid sequence

-Ser-Leu-Lys-Met-Ala-Asp-Pro-Asn-Arg-Phe-Arg-Gly-Lys-Asn-Leu-Pro-

R′ and R″ are alkyl groups containing 15 carbon atoms, R‴ is a cysteine residue and the adjuvant is alum.

Another preferred vaccine is an anti-rabies vaccine in which the immunologically active peptide-fatty acid conjugate has an amino acid sequence corresponding to a synethic peptide fragment designated N-V12b derived from the nucleoprotein of rabies virus which is recognized by a mouse monoclonal antibody and by human immune T cells. This fragment has the amino acid sequence
His-Phe-Val-Gly-Cys-Tyr-Met-Gly-Glu-Val-Arg-Ser-Leu-Asn-Ala-Thr-Val-Ile-Ala-Ala-Cys-Ala-Pro-His-Glu-

Such peptide-fatty acid conjugates may be prepared for example by adding a spacer of Gly-Gly-Lys to an N-terminal region of a virus glycoprotein which produces a T cell response and then adding two fatty acid side chains to the amines of the N-terminal lysine, the coupling of the fatty acid moieties being carried out by the symmetric anhydride method (Hopp, Molecular Immunology, 21: 13-16 (1984)).

According to a preferred form of the invention, the Gly-Gly-Lys spacer is added to an N-terminal region of fragment of the glycoprotein D molecule of HSV-1 and HSV-2 which produce a T cell response, following which a palmitic acid side chain is linked to each of the alpha and epsilon amino groups of the terminal lysine.

The N-terminal region of either gD-1 or gD-2 may comprise a synthetic peptide with sequence homology manually synthesized using Merrifield solid phase methods (Merrifield, J. Am. Chem. Soc. 85: 2194 (1963); Steward et al, (ed), Solid phase peptide synthesis, W.H. Freeman and Co., San Francisco, CA (1969)).

The resulting products are useful as anti-virus vaccines, particularly anti-HSV-1 and anti-HSV-2 vaccines. The process is applicable to viruses generally and particularly to other members of the herpes virus group.

As stated, it was discovered that the vaccines of the invention achieve significant protection for a protracted period of time against a large dose of virus by a single injection. Considering the fact that prior researchers generally concluded that high antibody titers are extremely important in providing protection against HSV-1 and HSV-2 infection, it was surprising that a protective immune response against HSV infection need not stimulate an antibody response. Rather it was discovered that with vaccines of this invention a T cell proliferative response in the absence of an antibody response leads to effective long term protection.

## Detailed Description of the Invention

The invention is described hereinafter in detail with respect to preparation and testing for immunogenicity of an anti-HSV-1 or HSV-2 vaccine comprising a synthetic peptide-fatty acid conjugate, the synthetic peptide having sequence homology with an N-terminal region or fragment of envelope glycoprotein D (gD) of either HSV-1 or HSV-2 which produces a T cell response. However, as previously noted the peptide portion of the conjugate may comprise a fragment of the nucleoprotein of rabies virus which is an immunodominant T cell antigenic determinant.

Considering the constituent parts of the vaccine, the HSV specific component of the peptide-fatty acid conjugate comprises an N-Terminal fragment of the surface glycoprotein D (gD), i.e. an amino acid peptide chain which produces a T cell response. The peptide may be the 23 amino acid peptide chain, the sequence of which was deduced from a terminal sequence of the gD molecule, a protein component of HSV-2 (Watson, Gene 158: 303 (1983)). The entire 23 amino acid sequence of the N-terminal fragment of HSV-2 gD is

(1) (2) (3) (4) (5) (6) (7)
$NH_2$-Lys-Tyr-Ala-Leu-Ala-Asp-Pro-
(8) (9)(10)(11)(12)(13)(14)(15)
Ser-Leu-Lys-Met-Ala-Asp-Pro-Asn-
(16)(17)(18)(19)(20)(21)(22)
Arg-Phe-ARg-Gly-Lys-Asn-Leu-
(23)
Pro-COOH

The 23 amino acid sequence of the N-terminal fragment of HSV-1 gD differs from the HSV-1gD in that alanine is at position 7 and aspartic acid is at position 21 (Dietzschold et al, J. of Viro., 52, No. 2: 431-435 (1984)).

Rather than using the entire 23 amino acid N-terminal protein of HSV-2 gD or HSV-1 gD, certain subunits thereof may be used provided they give a T cell response. A typical subunit of HSV-2 gD is

(8) (9)(10)(11)(12)(13)(14)
-Ser-Leu-Lys-Met-Ala-Asp-Pro-
(15)(16)(17)(18)
-Asn-Arg-Phe-Arg-
(19)(20)(21)(22)(23)
-Gly-Lys-Asn-Leu-Pro-

As previously stated the synthetic replicas of the above-noted N-terminal fragments may be prepared using solid phase methods (see Merifield, J. Am. Chem. Soc. 65: 2149 (1963); Stewart et al (ed), Solid phase peptide synthesis, W.H. Freeman and Co., San Francisco (169)).

There is added to the N-terminus of the peptide fragments a space for Gly-Gly-Lys$(NH_2)_2$. Such addition can be accomplished as part of the synthesis of the n-terminal peptide fragment. Preferably, cysteine is also added at the C-terminus for linkage to other "carrier" molecules, which are generally relatively large proteins.

There are then coupled to the alpha and epsilon amines of the lysine terminus saturated fatty acids generally having from about 11 to about 21 carbon atoms, examples of which are palmitic, stearic and oleic acids. The procedure by which conjugates of this general type are formed is well known (Hopp, Moleuclar Immunology. 21: 13-16 (1984)).

The peptide-fatty acid conjugate, prepared as above, is mixed with a liposome composition comprising a mixture of three lipids, namely phosphatidyl choline, cholesterol and lysophosphatidyl choline, following the method of Thibodeau (see Thibodeau et al, "Genetic variation among influenza viruses", Acad, Press, N.Y., London (1981, p. 587). The weight proportion of the three constituents of which the liposome is formed may vary considerably. Preferably, the proportion by weight of the three lipids is 16:2:1, respectively.

The peptide-fatty acid conjugate-containing liposome preparation is then mixed with an adjuvant, e.g. alum or complete Freund's adjuvant. Generally a weight ratio of 1:1 is used when the adjuvant is CFA and results in an emulsion which is available for use as a vaccine. In the case of alum, from about 4 to about 16 parts, preferably about 8 parts by weight per part of peptide ( in the absence of spacer and fatty acid) may generally be used.

The vaccine is administered in a dosage range of from about 100 to 300 μg, preferably about 150 μg, based on the weight of peptide per se (in the absence of spacer and fatty acid), in order to obtain the desired immunogenic T cell response protective against HSV-1 and HSV-2 virus. Usually the vaccine may be administered in a single dose and protection against a large dose of herpes virus is provided. However, a series of doses at intervals of several weeks or months followed, if necessary, by a booster dose at an interval of several months to several years may be administered if necessary. So used, the vaccine will produce in laboratory animals a T cell response protective against a herpes virus.

The following examples further illustrate the present invention without, however, limiting the same thereto.

Example 1

A peptide having the sequence homology of the 23 amino acid peptide chain, the sequence of which was deduced from a N-terminal gD molecule of HSV-2, having a spacer of Gly-Gly-Lys added to the N-terminus and cysteine added to the C-terminus, and having the following formula was prepared by Merrifield solid phase methods (Merrifield 1963; Stewart et al, 1969, supra)

$(NH_2)_2$Lys-Gly-Gly)[1]-Lys-Tyr-Ala-Leu-Ala-Asp-Pro-Ser-Leu-Lys-Met-Ala-Asp-Pro-Asn-Arg-Phe-Arg-Gly-Lys-Asn-Leu-Pro-(Cys)[2]COOH

More specifically the peptide molecule having the amino acid sequence immediately above, and sometimes referred to herein as "1-23(2)", was synthesized as follows:

All N-tert-butoxycarbonyl (BOC) amino acids were purchased from Sigma Chemical Co., BOC-Cys-O-Resin, L-t-Amyloxycarbonyl-N-Tosyl-L-Arginine, and L-BOC-O-Benzyl-L-Serine were purchased from Peninsula Laboratories, Inc. Peptides were manually synthesized using Merrifield solid phase methods (Merrifeld, 1963; Stewart, 1969, supra) with the following modifications.

(1) A series of three washes, the first with methylene chloride, the second with absolute ethanol and the third with methylene chloride was used instead of dioxane and chloroform before and after deprotection of N-t-BOC amino acid groups; (2) N-t-BOC amino acids were deprotected with 25% trifluoroacetic acid in methylene chloride; (3) completeness of the deprotection and coupling reactions was monitored using the color tests described by Kaiser et al, Annal. Biochemistry, 34: 595-598 (1970). After synthesis the resin was dried, and 50 equivalents of thioanisole were added. The side protection groups were removed and the peptide was cleaved from the resin with anyhydrous hydrogen floride. After removal of the anyhydrous hydrogen floride, the peptide resin mixture was extensively washed with ethyl acetate and ether to remove the thioanisole. The cleaved peptide was extracted with 1.5% $NH_4CO_3$ and lyophilized. The amino acid sequence of the peptide was verified by automated Edman degradation as described by Hunkapillar and Hood (Biochemistry 17: 2124-2133 (1978)).

Example 2

To add palmitic acid side chains, the N-terminal lysine was coupled as the bis-t-butyloxycarbonyl derivative, then deprotected using trifluoroacetic acid. The palmitic acid moieties were coupled by the symmetric anhydride method (Hopp, 1984, supra). Thus, the molecule is:

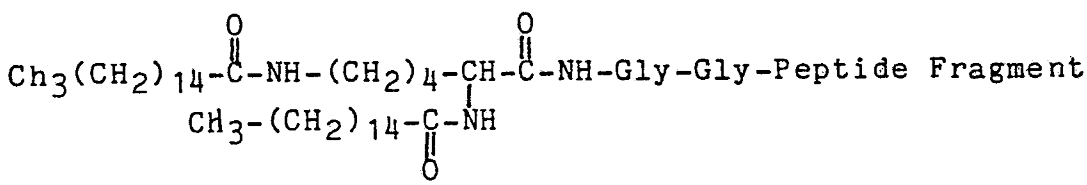

Example 3

The fatty acid-peptide conjugate of Example 2 was then mixed with a liposome comprising 3 lipids as follows (see Thibodeau et al, 1981)):

Phosphatidyl choline, cholesterol, and lysolecithin were each dissolved in MeOH/chloroform (1:3) and then mixed in the ratio of 16:2:1, respectively. This mixture was then blown down with $N_2$ rotating the vial in warm $H_2O$ to get an even film over the entire vial. Five mg. of the peptide-palmitic acid conjugate was dissolved in 2 ml of a 1% octylglycoside in phosphate buffered saline solution (PBS). Ten mg. of the lipid mixture was then added to this peptide solution. Dialysis was carried out against PBS using a 3500 dalton cutoff Spectropore dialysis mebrane for 24 hours. The liposomes were sonicated for 5 minutes.

Example 4

Testing of the vaccine

The first experiment was done with a single injection of vaccine into the two hind footpads of a group of six Balb/c mice. The volume given was approximately 0.2 ml/animal or 10 µg/g body weight. In addition, as controls Balb/c mice (6 mice/group) were immunized with 1-23(2) peptide in CFA, CFA alone, and UV-inactivated HSV-1 in CFA, as follows:

(1) 1-23(2) at 100 µg/animal;

(2) UV-inactivated HSV-1 at 10 PFU/animal;

(3) Vaccine of Example 3 (1-23(2)-palmitic acid-liposome, CFA) at approximately 150 µg peptide/ani-

[1] A spacer group added to the peptide and to which palmitic acid side chains are added to the Lys amino groups.
[2] A cysteine amino acid used for linkage to other carrier molecules.

mal;

(4) CFA alone.

Six and 1/2 months after this single immunization, the mice were challenged with a lethal dose of HSV-2 ( a 10 $LD_{50}$ dose of strain 186 grown in BHK cells which are mouse fibroblasts) in both hind footpads and the animals were examined for the next 30 days for paralysis and death. The results are plotted in Figure 11. It can be seen that by day 8, many of the animals were symptomatic. Only two groups, the HSV-1-primed (-♦-♦-) and the 1-23(2) palmitic acid-liposome-CFA-primed (-□-□-) animals, appeared normal. On day 18, when all of the control animals had died (CFA-◇-◇-, 1-23(2) (-■-■-), the HSV-1 and 1-23(2)-palmitic acid-liposome, CFA vaccine immunized animals survived. At day 120, 33% of both of those groups were normal.

Example 5

To determine the mechanism of protection, the antibody response (humoral response in terms of antibody which can bind the peptide to virus and which can neutralize the activity of virus infectivity in an in vetro assay) was studied. T cells responses, by measuring the ability of T cells to respond to antigen by their production of lymphokines after antigen simulation were also determined. First, since it is known that neutralizating antibody can protect animals from an HSV infection (Balachandran, 1982 supra; Dix, et al, Infec. and Immun., 34: 192-199 (1981) Kapoor et al, J. Gen, Virol., 60: 225-233 (1982)), an effort was made to determine if this was the cause of the protection seen.

Animals were bled 1 week after HSV-2 challenge sonce specific antibody which had already been induces by previous exposure to antigen (peptide) should at that time have been high, and serum was taken from animals and frozen until time of assay. The results obtained as set forth in Table 5, below.

Table 5

Neutralization Titers of Anti-HSV Antibody

| Pooled Serum* From Each Group | Neutralizing Antibody for HSV-1 | HSV-2 |
|---|---|---|
| 1. Control | 12** | 6 |
| 2. HSV-1 | 389 | 97 |
| 3. 1-23(2) | 6 | 8 |
| 4. Vaccine of Example 3 (1-23(2)-liposome CFA) | 5 | 5 |

* Animals were challenged with HSV-2 in footpads 6 and 1/2 months after a single immunization of antigen in CFA. Bleedings were done at 1 week after challenge with HSV-2. 100 PFU of virus in 25 microliters were added to serum antibody in the same volume with the serum being diluted in two fold dilutions. This mixture in 96 well Costar plates was incubated for 1 hour at 37° C and BHK cells were added at a concentration of 5 x $10^4$ cells/ml in 50 microliters. Three to four days later, the cells were stained with crystal violet dissolved in 10% buffer formalin.

** Titer=1/dilution of sample x $2^x$; x=well number

As shown in Table 5, neutralizing antibody titers could explain the protection in the HSV-1 group, but could not explain the protection seen with the group immunized with the vaccine of Example 3 (1-23(2)-liposome CFA).

Example 6

This example pertains to detection of anti-viral binding activity and should be considered in connection with Figures 12(a) and 12(b). Balb/c mice were challenged with HSV-2 in the hind footpads 6 and 1/2 months after single immunization with the vaccine of Example 3. Bleedings were done 1 week after challenge with HSV-2. A radioimmunoassay was carried out using HSV-1 (-○-○-) and HSV-2 (-Δ-Δ-) infected and uninfected (-■-■-) BHK lysates, and 1-23(H) peptides (-●-●-) and phosphate buffered saline alone (-∇-∇-) as immunoadsorbants. The assay was done by (a) preincubation of vinyl 96 well culture plates with the various immunoadsorbants in saline overnite at 4°C; (b) coating with 50% Fetal Calf Serum; (c) incubation of the Balb/c antisera for 3 hours at room temperature, and (d) labeling with 5000 CPM of $^{125}$I-rabbit Fab anti-mouse Ig. Figures 2(a) and 2(b) show the amount of radioactively labeled anti-mouse antibody bound versus the dilution of the antiserum tested. The highest concentration shown is considered the nonspecific binding region. As shown by the figures, anti-peptide binding activity at the highest concentration tested could be detected when the animals have been immunized with 1-23(2)-lipsome (A) and 1-23-(2) (c), but not with HSV-1 (B) or CFA (D). The nature of the binding activity is unclear since it is detected in the non-specific part of the titration curve. However, it is clear that antibody to the virus was detected only in animals immunized with

SHV-1 (B).

In view of these experiments it was concluded that the vaccine of the invention induces no detectable antibody which can bind the virus.

Example 7

T cells from animals immunized with the vaccine of Example 3 were obtained from the lymph nodes, purified on nylon wool columns (Julius, et al, Europ. J. Immuno. 3: 645 (1977)), and then tested for responsiveness in vitro by T cell proliferation measured through the incorporation of $^{3}H$-thymidine into DNA after three days in culture (Corradin, et al, J. Immuno. 119: 1046 (1977)). The results appear in Table 6.

TABLE 6

| | |
|---|---|
| T cells alone | 7,300 CPM |
| T cells + 1-23(2) peptide 50 $\mu$g/ml | 30,400 CPM |
| T cells + HSV-1 ($10^6$ PFU/ml) | 20,300 CPM |

The data in Table 5 show that T cells from animals vaccinated respond to the peptide and also cross-react with the virus. The T cell proliferation test in vitro has generally been accepted as indicative of the presence of an antigen specific T cell response as correlating with T cell effector function. T cells induced by the peptide-containing vaccine are responsive not only to the specific peptide but also to a corresponding exiologic agent of the herpes virus infection, i.e. HSV-1.

Example 8

A further experiment was carried out in the same manner as Example 4. Animals were immunized in the hind footpads with the following: (a) 1-23(2)-palmitic acid-liposomes in CFA (13 animals) (-□-□-); (b) UV-inactivated HSV-1 in CFA (5 animals) (-□-□-); (c) CFA alone (10 animals) (-◇-◇-); (d) liposome in CFA (11 animals) (-◆-◆-); and (e) 1-23(2)-palmitic acid-liposome in saline (5 animals) (-■-■-). The animals were then challenged 3 and 1/2 months after immunization with a 4 LD 50 of HSV-2(186). The results are presented in Figure 13.

Examples 9 and 10

The procedures of Examples 1 and 2 were repeated with the exception that the adjuvant used in Example 10 was MDP-Thr, the threnoyl analogue of muramyl dipeptide (N-acetylmuramyl-L-threonyl-D-isoglutamine, a formulation which selectively elicits the formation of antibodies of protective isotypes and of cell mediated immunity (Allison et al, J. of Immunol. Methods, 95: 157-168 (1986)).

Example 11

Testing of the vaccine

The experiment was done with a single injection of vaccine into the two hind footpads of a group of ten Balb/c mice. The volume given was approximately 0.2 ml/animal or 10 µg/g body weight. In addition, as controls Balb/c mice (10 mice/group) were immunized with the vaccine 1-23(2)-palmitic acid - lipsome in CFA or unimmunized as follows:

(1) Vaccine of Example 11 (1-23(2)-palmitic acid-liposome-Thr-MDP as adjuvant) 150 µg peptide/animal;

(2) Vaccine of Example 3 (1-23(2)-palmitic acid-liposome, CFA) at approximately 150 µg peptide/animal;

(3) Unimmunized.

Three months after this single immunization, the mice were challenged with a letal dose of HSV-2 ( a 10 $LD_{50}$ dose of strain 186 grown in BHK cells which are mouse fibroblasts) in both hind footpads and the animals were examined for the next 30 days for paralysis and death. The results are plotted in Figure 14. It can be seen that by day 8, many of the animals were symptomatic. Two groups, the 1-23(2) palmitic acid-liposome-Thr-MDP-primed animals (-◇-◇-) and the 1-23(2) palmitic acid-liposome-CFA-primed (-■-■-) animals, appeared normal. On day 15, when all of the control animals had died, almost all of the immunized animals were protected.

Example 12

A peptide having the sequence homology of the 25 amino acid peptide chain of rabies nucleoprotein, the sequence of which was determined as described above, having a spacer of Gly-Gly-Lys added to the N-terminus and cysteine added to the C-terminus, and having the following formula was prepared by Merrifield

solid phase methods (Merrifield 1963; Stewart et al, 1969, supra)

$(NH_2)_2$Lys-Gly-Gly)[3]-His-Phe-Val-Gyl-Cys-Try-Met-Gly-Glu-Val-Arg-Ser-Leu-Asn-Ala-Thr-Val-Leu-Ala-Ala-Cys-Ala-Pro-His-Glu-(Cys)[4]COOH

More specifically the peptide molecule having the amino acid sequence immediately above, and sometimes referred to herein as "N-V12(b)", was synthesized as follows:

All N-tert-butoxycarbonyl (BOC) amino acids were purchased from Sigma Chemical Co., BOC-Cys-O-Resin, L-t-Amyloxycarbonyl-N-Tosyl-L-Arginine, and L- BOC-O-Benzyl-L-Serine were purchased from Peninsula Laboratories, Inc. Peptides were manually synthesized using Merrifield solid phase methods (Merrifield, 1963; Stewart, 1969, supra) with the following modifications.

(1) A series of three washes, the first with methylene chloride, the second with absolute ethanol and the third with methylene chloride was used instead of dioxane and chloroform before and after deprotection of N-t-BOC amino acid groups; (2) N-t-BOC amino acids were deprotected with 25% trifluoroacetic acid in methylene chloride; (3) completeness of the deprotection and coupling reactions was monitored using the color tests described by Kaiser et al, Annal. Biochemistry, 34: 595-598 (1970). After synthesis the resin was dried, and 50 equivalents of thioanisole were added. The side protection groups were removed and the peptide was cleaved from the resin with anyhydrous hydrogen floride. After removal of the anyhydrous hydrogen floride, the peptide resin mixture was extensively washed with ethyl acetate and ether to remove the thioanisole. The cleaved peptide was extracted with 1.5% $NH_4CO_3$ and lyophilized. The amino acid sequence of the peptide was verified by automated Edman degradation as described by Hunkapillar and Hood (Biochemistry 17: 2124-2133 (1978)).

Example 13

To add palmitic acid side chains, the N-terminal lysine was coupled as the bis-t-butyloxycarblnyl derivative, then deprotected using trifluoroacetic acid. The palmitic acid moieties were coupled by the symmetric anhydride method (Hopp, 1984, supra). Thus, the molecule is:

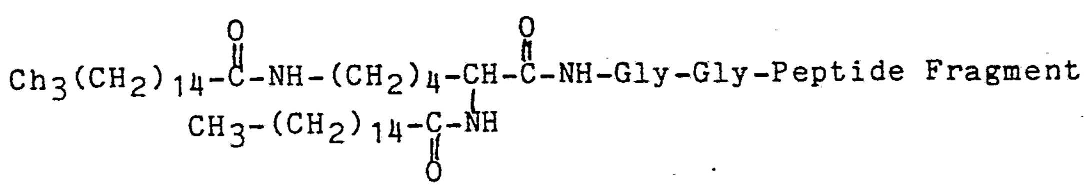

where the peptide fragment is that of N-V12b (see Table 2, infra. p 52 )).

Example 14

The fatty acid-peptide conjugate of Example 13 was then mixed with a liposome comprising 3 lipids as follows (see Thibodeau et al, 1981)):

Phosphatidyl choline, cholesterol, and lysolecithin were each dissolved in MeOH/chloroform (1:3) and then mixed in the ratio of 16:2:1, respectively. This mixture was then blown down with $N_2$ rotating the vial in warm $H_2O$ to get an even film over the entire vial. Five mg. of the peptide-palmitic acid conjugate was dissolved in 2 ml of a 1% octylglycoside in phosphate buffered saline solution (PBS). Ten mg. of the lipid mixture was then added to this peptide solution. Dialysis was carried out against PBS using a 3500 dalton cutoff Spectropore dialysis mebrane for 24 hours. The liposomes were sonicated for 5 minutes.

Example 15

Testing of the vaccine

The first experiment was done with a single injection of vaccine into the two hind footpads of a group of eight Balb/c mice. The volume given was approximately 0.2 ml/animal or 10 µg/g body weight. In addition, as controls Balb/c mice (10 mice) were immunized with:

(1) CFA alone;

(2) Vaccine of Example 14 (N-V12(b)-palmitic acid-liposome, CFA) at approximately 10 µg peptide/animal;

One month after this single immunization, the mice were challenged with a letal dose of rabies virus (a 2 $LD_{50}$ dose of strain CVS-24 grown in BHK cells which are mouse fibroblasts) and the animals were examined for the next 21 days for paralysis and death; 87% of vaccine injected animals survived.

[3] A spacer group added to the peptide and to which palmitic acid side chains are added to the Lys amino groups.
[4] A cysteine amino acid used for linkage to other carrier molecules.

Table 7

IM Challenge with 2 $LD_{50}$ of CVS-24

4 Weeks After Immunization

| Vaccine | Mortality* |
|---|---|
| N-V12b-Liposome-CFA | 1/8 |
| None | 7/10 |

*Mortality determined 3 weeks after challenge

The published articles identified in the foregoing specification are incorporated by reference herein in their entirety.

TABLE 1

$NH_2$-Terminal Amino Acid Sequences Antigenically Active Fragments of ERA N and NS Proteins

N Protein

| Fragment # | MAb # | $NH_2$-aa sequences |
|---|---|---|
| N-W1 | 377, 802<br>805, 816 | $NH_2$-Terminus block |
| N-W2 | 377, 802 | NT |
| N-W3 | 377, 802 | 90 99<br>TSYGIVIARK |
| N-W4 | 377, 802 | 198 210<br>STIPNFRFLAGTY |
| N-C1 | 377,802 | 339 351<br>SVLGGYLGEEFFG |
| N-V10 | 377-802 | 374 390<br>LTKTDVALADDGTVNSD |
| N-V12 | 816 | 184 195<br>a, HHTLMTTHKMCA |
| | | 279 291<br>b, TAVPHSYFIHFRS |
| **NS Protein** | | |
| NS-V8 | 701, 721 | 75 98<br>GKYREDFQMDEGEDPSLLFQSYLE |

TABLE 2

Synthetic Peptides Corresponding to Antigenic Regions of N and NS

**1. Region N-V10**

| Peptide | Sequence |
|---|---|
| N-V10a: | 383 – 393<br>DDGTVNSDDE |
| N-V10b: | 374 – 392<br>LTKTDVALADDGTVNSDDE |
| N-V10c-L: | 369 – 383<br>$NH_2$-YEAAELTKSDVALAD |
| N-V10c-S | 369 – 383<br>$NH_2$-YEAAELTKSDVALAD |

**2. Region N-V12**

| | |
|---|---|
| Peptide N-V12a: | 184 – 203<br>$NH_2$-HHTLMTTHKMCANWSTIPNP |
| Peptide N-V12b | 313 – 337<br>$NH_2$-HFVGCYMGQVRSLANATVIAACAPHE |

**3. Region NS-V8**

| | |
|---|---|
| Peptide NS-V8 | 75 – 90<br>$NH_2$-GKYREDFQMDEGDPS |

TABLE 3

ANTIGEN-INDUCED T CELL PROLIFERATION BY RABIES NUCLEOCAPSID AND SYNTHETIC PEPTIDES*

| Antigen conc. [μg/ml] | N-V12b | N-V10c | Antigen NS-V8 | ERA N |
|---|---|---|---|---|
| 25 | 3,683 | 1,716 | 1,732 | 9,890 |
| 12 | 2,721 | 2,104 | 1,102 | 7,406 |
| 6 | 2,956 | 2,175 | 1,595 | 5,760 |
| 3 | 3,122 | 1,389 | 1,023 | 4,258 |
| 1.5 | 3,640 | 1,484 | 944 | 3,502 |
| 0.8 | 2,436 | 909 | 562 | 2,902 |
| 0.4 | 3,146 | 974 | 651 | 1,979 |
| Medium | 407 | 483 | 463 | 407 |

* T cell proliferation in the presence of antigen-presenting cell populations of the same HLS-Dr type was determined in the presence and absence of antigen in triplicate samples. The mean cpm are represented, and in all cases the standard errors of the means were below 10% the value of the means.

TABLE 4

Reactivity of MABs and Antipeptide Sera
With Different Rabies Virus Strains

| Antibody | Mokola 4 | Duvenhage 1 | Duvenhage 4 | CVS | HEP | ERA | PM |
|---|---|---|---|---|---|---|---|
| 377-2 | 0 | 0 | + | 0 | + | + | + |
| 802-2 | + | + | + | + | + | + | + |
| 805-3 | + | + | + | + | 0 | + | + |
| 816-1 | + | + | + | + | 0 | + | + |
| 701-5. | 0 | 0 | 0 | + | + | + | + |
| 721-2 | 0 | 0 | 0 | + | + | + | + |
| anti-N-V10b | + | + | + | + | + | + | + |
| anti-N-V10c | + | + | + | + | + | + | + |
| anti-NS-V8 | 0 | 0 | 0 | + | + | + | + |

+, positive fluorescense staining; 0, no staining

## Claims

1. A vaccine for generating an immunogenic T cell response protective against a virus disease state, said vaccine comprising (1) a peptide-fatty acid conjugate having the formula:

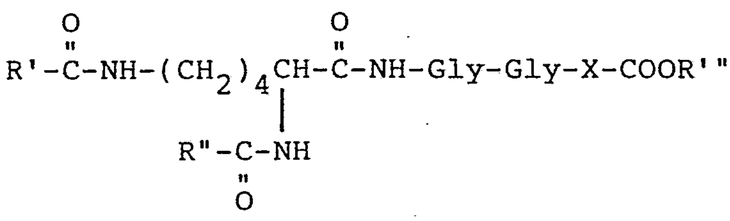

where R′ and R″ are alkyl groups containing 5 to 30 carbon atoms, and R‴ is selected from the group consisting of hydrogen and at least one amino acid residue, and X is an amino acid sequence corresponding to that of a peptide fragment derived from a protein of the virus which produces a T cell response, or a synthetic replica of said fragment, (2) a lipsome composition comprising a mixture of phosphatidyl choline, cholesterol and lysophosphatidyl choline, and (3) an adjuvant, characterised in that the vaccine is directed against rabies virus and the virus protein is the nucleoprotein of rabies virus which is an immunodominant T cell antigenic determinant.

2. A vaccine according to claim 1 in which each of R′ and R″ is an alkyl group containing from 10 to 20 carbon atoms, R‴ is a cysteine residue, and the adjuvant is selected from the group consisting of alum, complete Freund's adjuvant, and MDP-Thr.

3. A vaccine according to claim 1 in which each of R′ and R″ is an alkyl group containing 15 carbon atoms, and R‴ is a cysteine residue.

4. A vaccine according to any of claims 1 to 3 in which said adjuvant is alum gel or MDP-Thr.

5. A vaccine according to any of claims 1 to 4 in which said phosphatidyl choline, cholesterol and lysophosphatidyl choline are present in the proportions by weight of 16:2:1, respectively.

6. A vaccine according to any of claims 1 to 5 in which X is:

-His-Phe-Val-Gly-Cys-Tyr-Met-Gly-Glu-Val-Arg-Ser-Leu-Asn-Ala-Thr-Val-Ile-Ala-Ala-Cys-Ala-Pro-His-Glu-

7. A vaccine according to any of claims 1 to 5 in which X is:

Tyr-Glu-Ala-Ala-Glu-Leu-Thr-Lys-Thr-Asp-Val-Ala-Lys-Ala-Asp-

each of R′ and R″ is an alkyl group containing 15 carbon atoms, R‴ is a residue of cysteine, and the adjuvant is alum gel or MDP-Thr.

8. A peptide-fatty acid conjugate having the formula:

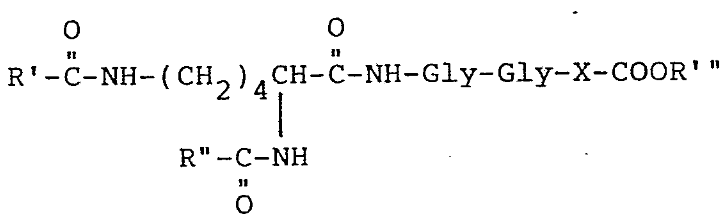

where R′ and R″ are alkyl groups containing 5 to 30 carbon atoms, R‴ is selected from the group consisting of hydrogen and at least one amino acid residue, and X is an amino acid sequence corresponding to that of a peptide fragment derived from the nucleoprotein of rabies virus which is an immunodominant T cell antigenic determinant, or a synthetic replica of said fragment.

9. A conjugate according to claim 8 in which each of R′ and R″ is an alkyl group containing from 10 to 20 carbon atoms, and R‴ is a cysteine residue.

10. A conjugate according to claim 8 or 9 in which each of R′ and R″ is an alkyl group containing 15 carbon atoms, and R‴ is a cysteine residue.

11. A conjugate according to any of claims 8 to 10 in which the peptide fragment has the amino acid

sequence:

-His-Phe-Val-Gly-Cys-Tyr-Met-Gly-Glu-Val-Arg-Ser-Leu-Asn-Ala-Thr-Val-Ile-Ala-Ala-Cys-Ala-Pro-His-Glu-

12. A composition useful for forming a vaccine for generating an immunogenic T cell response protective against rabies virus disease state when combined with an adjuvant, comprising an immunologically effective amount of (1) a peptide-fatty acid conjugate according to any of claims 8 to 11, and (2) a liposome composition comprising a mixture of phosphatidyl choline, cholesterol and lysophosphatidyl choline.

M 30·07·88

0290246

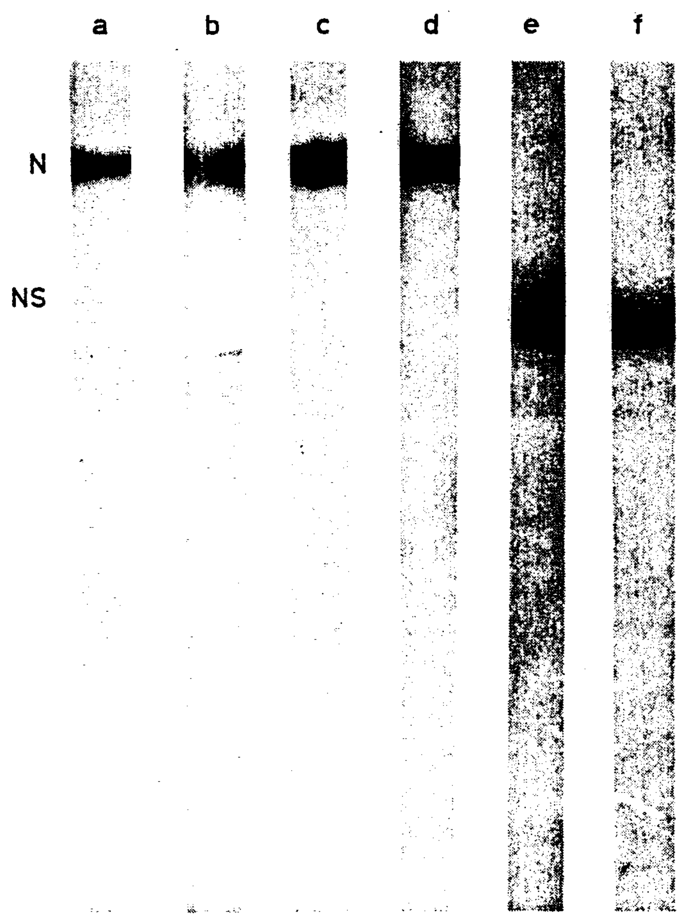


Figure 1

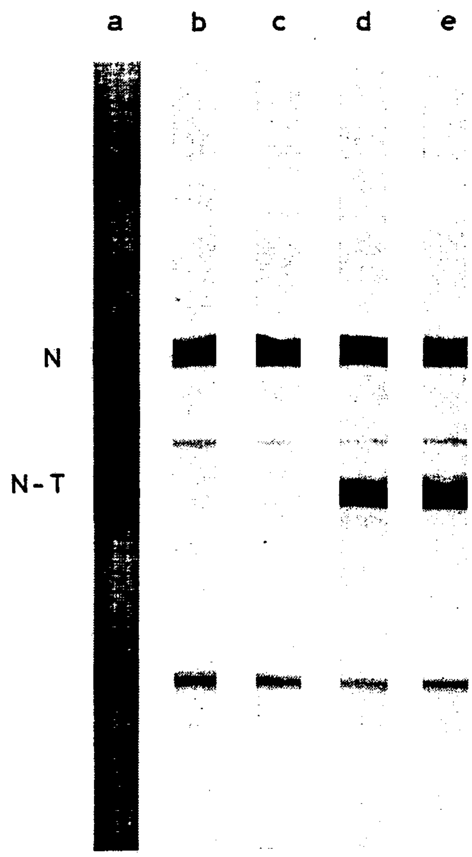


Figure 2

M 30.07.88

0290246

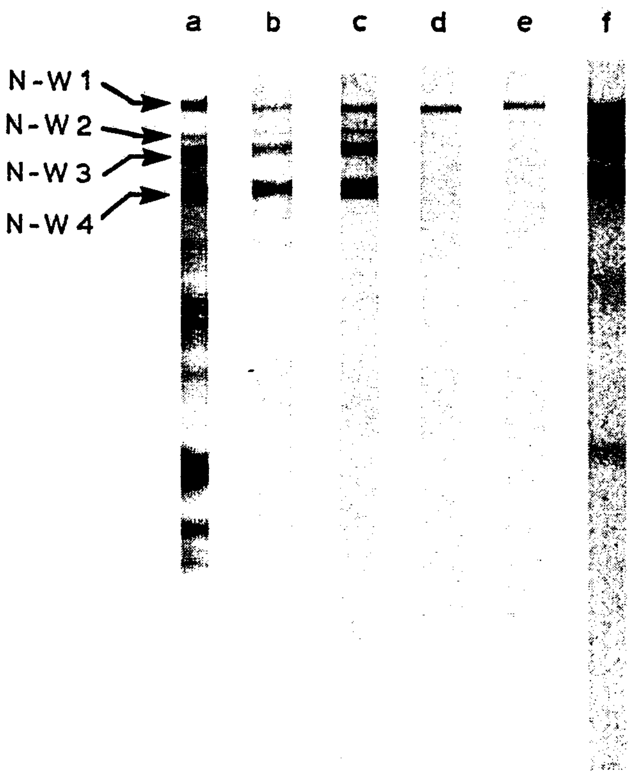


Figure 3

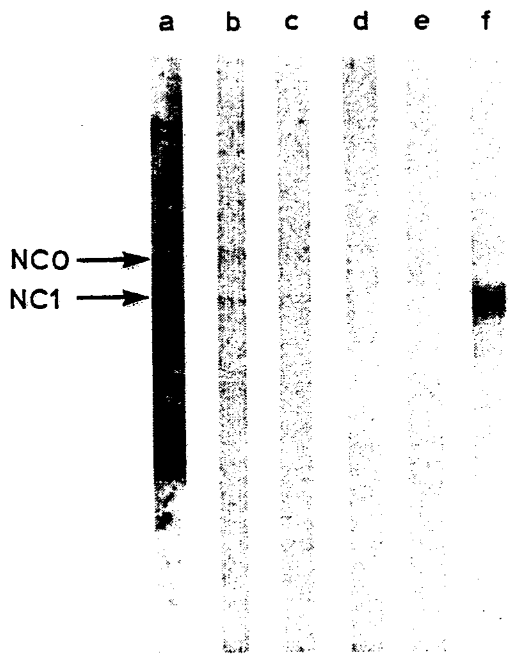


Figure 4

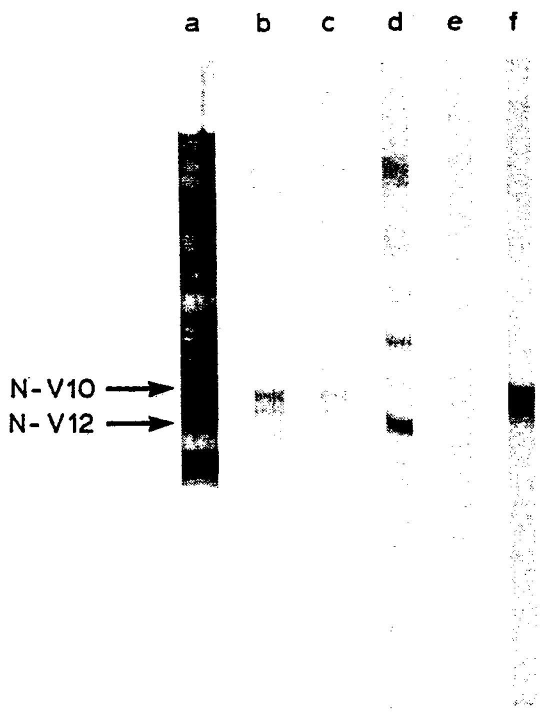


Figure 5

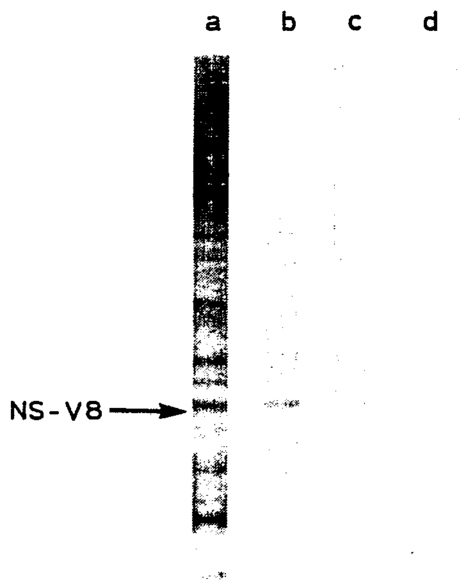


Figure 6

M 30·07·88

0290246

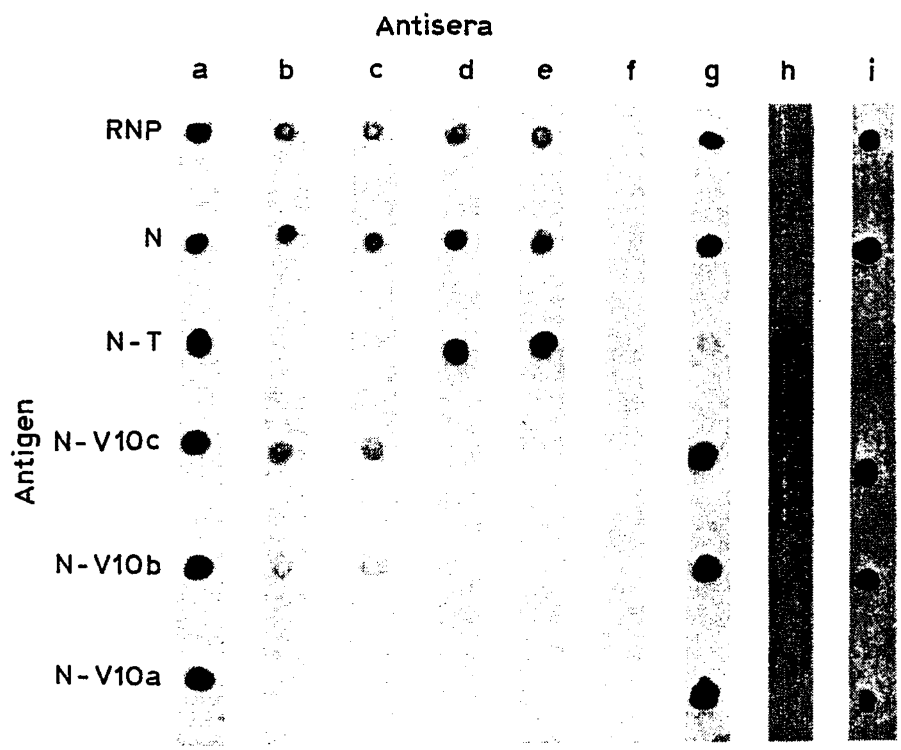


Figure 7

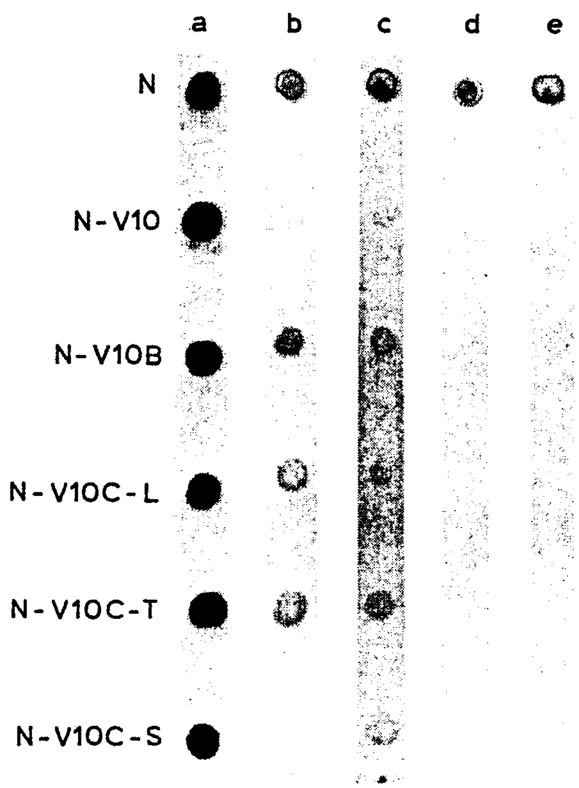


Figure 8

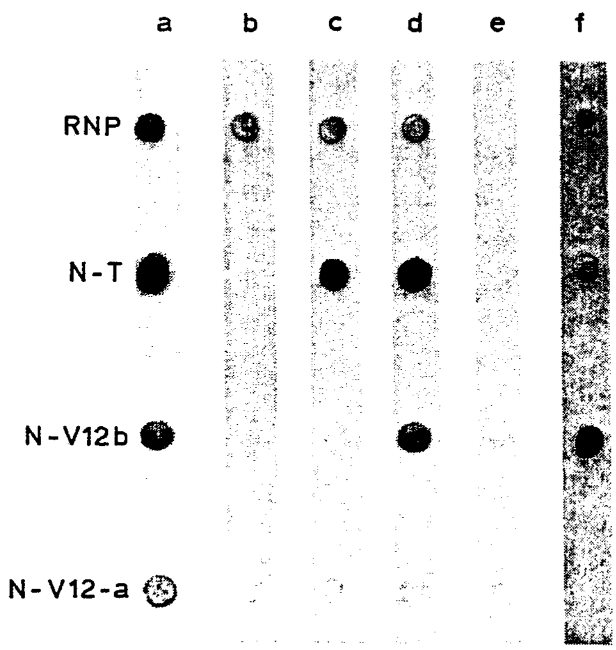


Figure 9

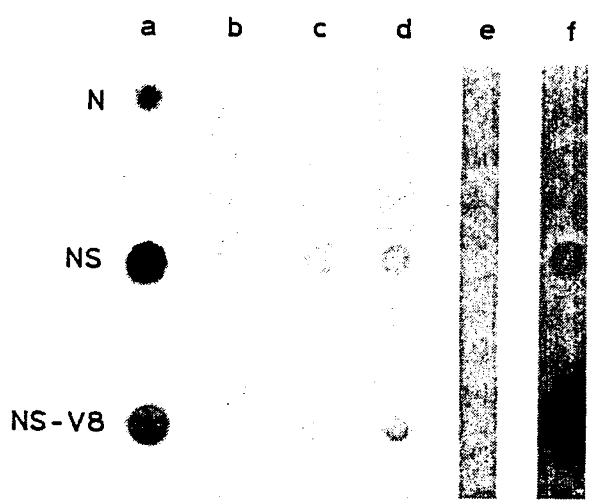


Figure 10

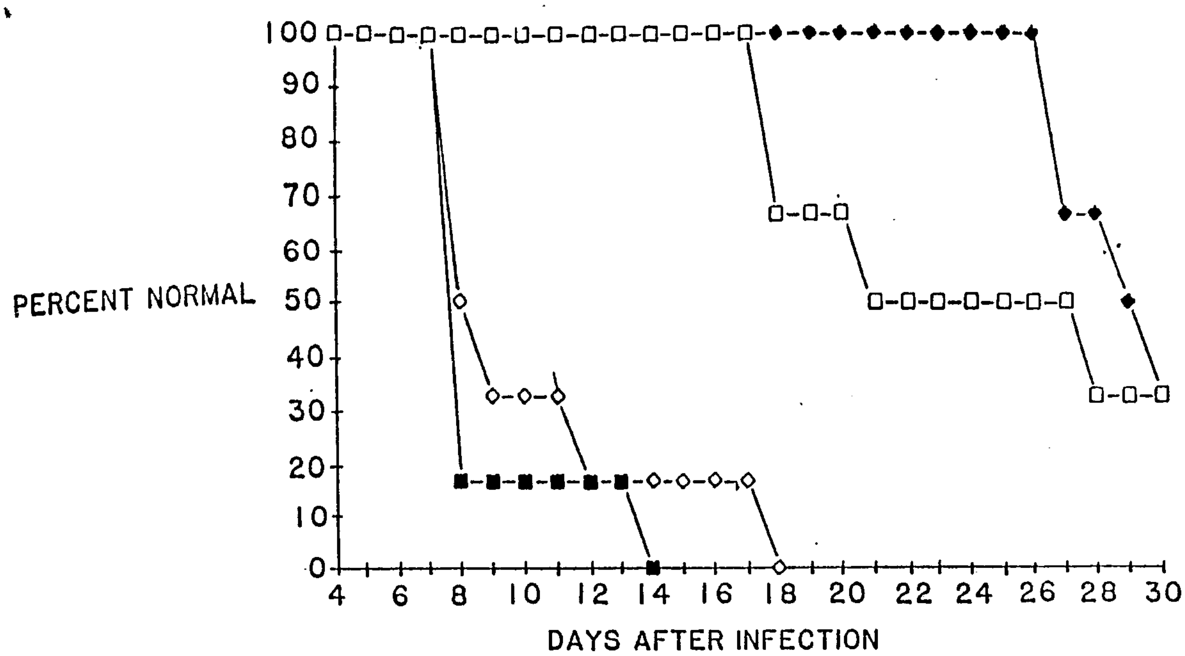
PERCENT NORMAL
100
90
80
70
60
50
40
30
20
10
0
4 6 8 10 12 14 16 18 20 22 24 26 28 30
DAYS AFTER INFECTION

FIG. 11

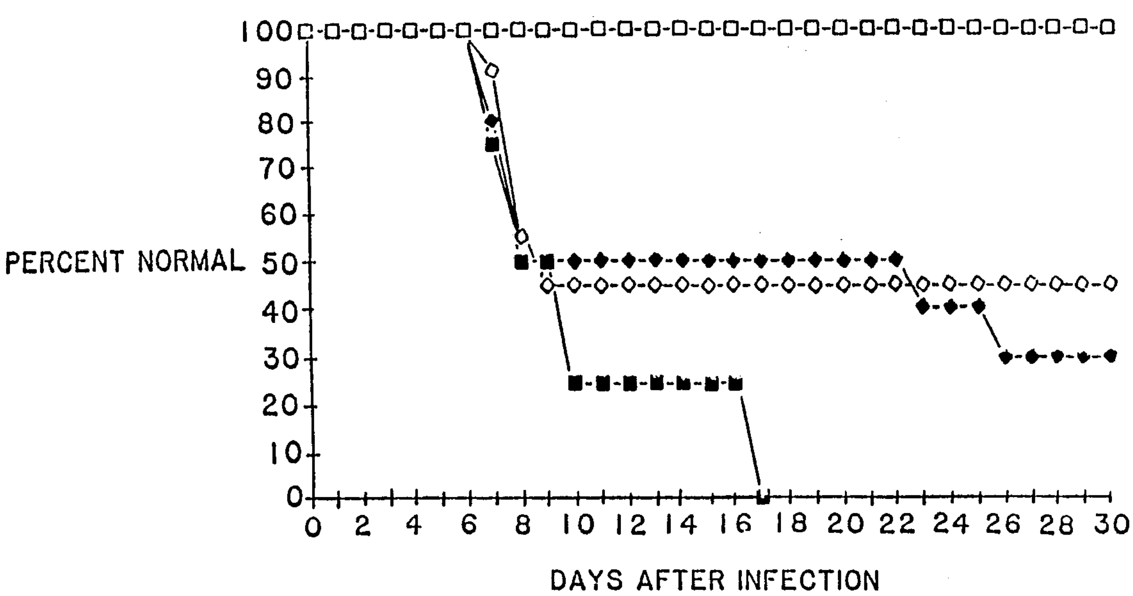
PERCENT NORMAL
100
90
80
70
60
50
40
30
20
10
0
0 2 4 6 8 10 12 14 16 18 20 22 24 26 28 30
DAYS AFTER INFECTION

FIG. 13

FIG. 12A

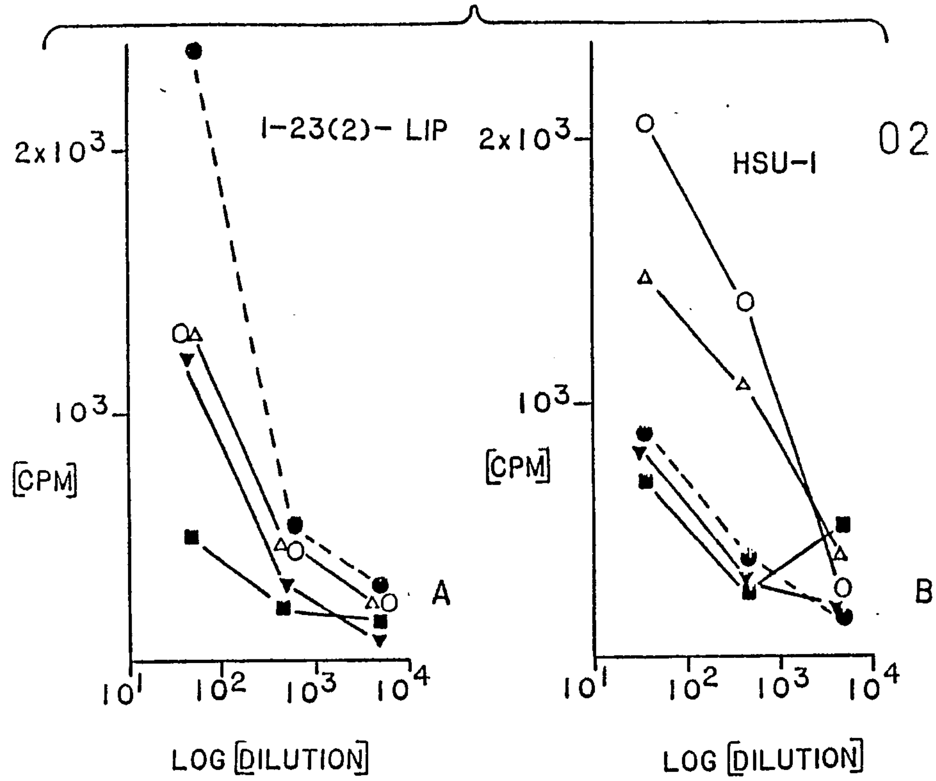


FIG. 12B

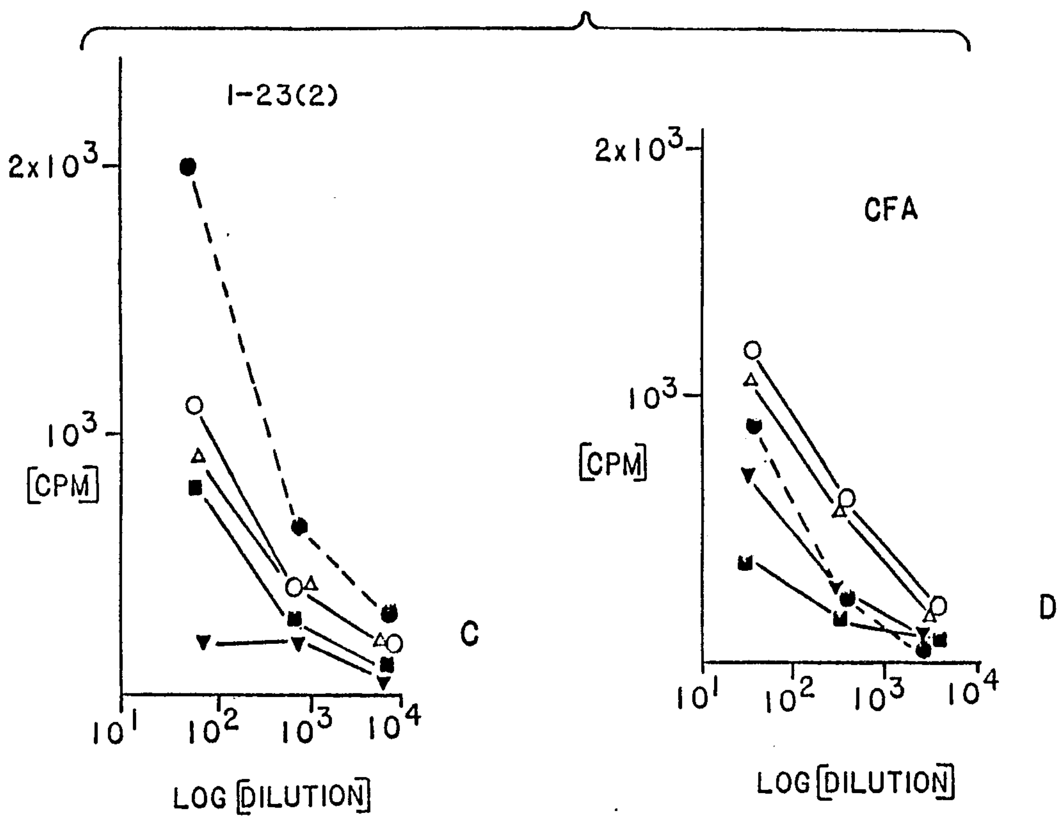

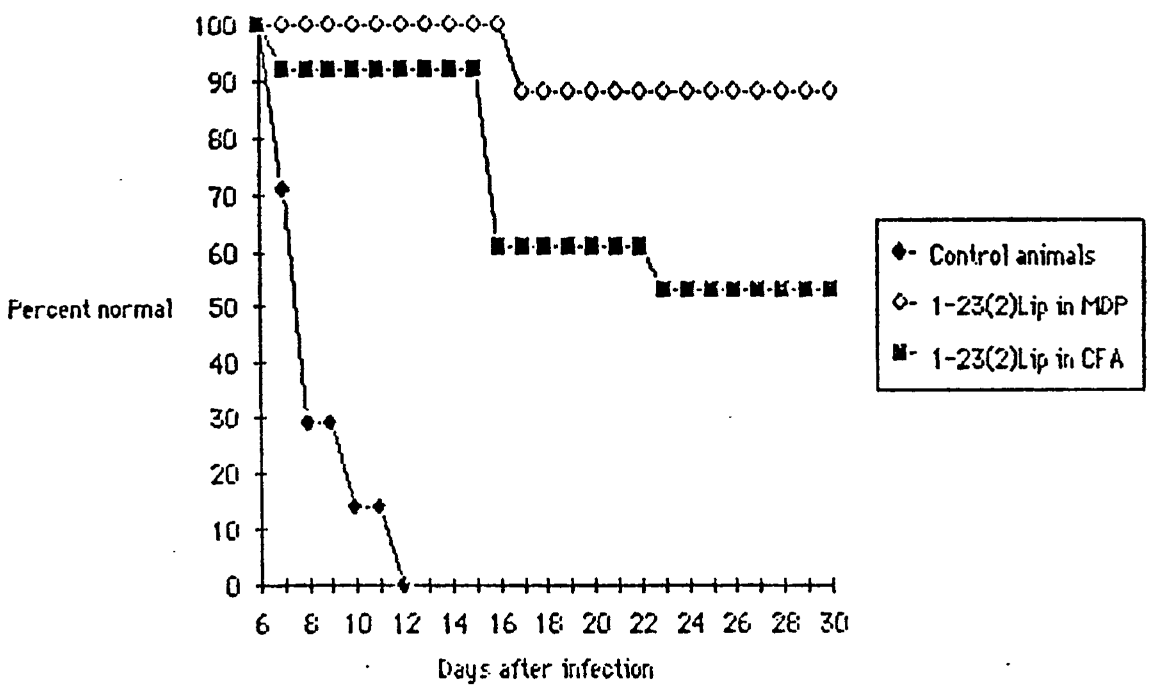
100
90
80
70
60
50
40
30
20
10
0
Percent normal
6 8 10 12 14 16 18 20 22 24 26 28 30
Days after infection
Control animals
1-23(2)Lip in MDP
1-23(2)Lip in CFA

Figure 14